(19) Europäisches Patentamt
European Patent Office
Office européen des brevets

(11) Veröffentlichungsnummer: **0 007 506**
**B1**

# (12) EUROPÄISCHE PATENTSCHRIFT

(45) Veröffentlichungstag der Patentschrift: **18.11.81**

(51) Int. Cl.³: **C 07 D 249/08,** C 07 D 233/64, A 01 N 43/50, A 01 N 43/64

(21) Anmeldenummer: **79102343.5**

(22) Anmeldetag: **09.07.79**

## (54) Alpha-Azolyl-keto-Derivate, Verfahren zu ihrer Herstellung und ihre Verwendung als Fungizide.

(30) Priorität: **21.07.78 DE 2832234**

(43) Veröffentlichungstag der Anmeldung: **06.02.80 Patentblatt 80/3**

(45) Bekanntmachung des Hinweises auf die Patenterteilung: **18.11.81 Patentblatt 81/46**

(84) Benannte Vertragsstaaten: **AT BE CH DE FR GB IT NL SE**

(56) Entgegenhaltungen:
CH-A-564 543
DD-A-117 460
DE-A-2 201 063
DE-A-2 324 010
DE-A-2 325 156
DE-A-2 431 073
DE-A-2 726 043
DE-A-2 734 365

(73) Patentinhaber: **BAYER AG, Zentralbereich Patente, Marken und Lizenzen, D-5090 Leverkusen 1, Bayerwerk (DE)**

(72) Erfinder: **Kranz, Eckart, Dr., Am Acker 9, D-5600 Wuppertal 1 (DE)**
Erfinder: **Krämer, Wolfgang, Dr., Am Eckbusch 39/162, D-5600 Wuppertal 1 (DE)**
Erfinder: **Büchel, Karl Heinz, Prof. Dr., Bergerheide 62, D-5600 Wuppertal 1 (DE)**
Erfinder: **Brandes, Wilhelm, Dr., Eichendorffstrasse 3, D-5653 Leichlingen 1 (DE)**
Erfinder: **Frohberger, Paul-Ernst, Dr., Willi-Baumeister-Strasse 5, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



EP 0 007 506 B1

# α-Azolylketoderivate, Verfahren zu ihrer Herstellung und ihre Verwendung als Fungizide

Die vorliegende Erfindung betrifft neue α-Azolylketoderivate, mehrere Verfahren zu ihrer Herstellung und ihre Verwendung als Fungizide.

Es ist bereits bekannt geworden, daß substituierte Phenoxytriazolylketo bzw. -hydroxyderivate im allgemeinen sehr gute fungizide Eigenschaften aufweisen [vergleiche DE-AS 2 201 063 (Le A 14118) bzw. DE-OS 2 324 010 (Le A 14 971)]. Die Wirkung ist jedoch, insbesondere bei niedrigen Aufwandmengen und -konzentrationen, in einigen Anwendungsbereichen nicht immer voll befriedigend.

Es wurden neue α-Azolylketoderivate der allgemeinen Formel

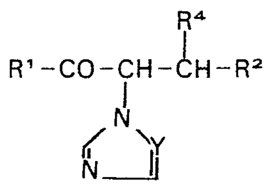 (I)

in welcher

$R^1$ für Alkyl mit 1 bis 4 Kohlenstoffatomen steht, welches gegebenenfalls substituiert sein kann durch Halogen, Alkylcarbonyloxy mit 1 bis 4 Kohlenstoffatomen im Alkylteil, durch Alkylsulfonyloxy mit 1 bis 4 Kohlenstoffatomen, durch Phenylcarbonyloxy- und Phenylsulfonyloxygruppen, wobei letztere noch gegebenenfalls am Phenyl durch Halogen und Alkyl mit 1 bis 2 Kohlenstoffatomen substituiert sein können, durch Alkoxy mit 1 bis 4 Kohlenstoffatomen und ferner noch durch Phenoxy, welches gegebenenfalls noch durch Halogen und Alkyl mit 1 bis 2 Kohlenstoffatomen substituiert sein kann, und endlich noch für Phenyl steht, welches gegebenenfalls substituiert sein kann durch Halogen, Cyano, Nitro und Alkyl mit bis zu 4 Kohlenstoffatomen, ferner durch Cyclohexyl, Halogenalkyl mit bis zu 2 Kohlenstoff- und bis zu 5 Halogenatomen und schließlich durch gegebenenfalls durch Fluor, Chlor, Cyano oder Nitro substituiertes Phenyl, Phenoxy oder Benzyl,

$R^2$ für die Gruppierung $-CX^1X^2R^3$ und für Alkoxycarbonyl mit 1 bis 4 Kohlenstoffatomen im Alkoxyteil steht, wobei

$R^3$ für Halogen, Halogenalkyl mit 1 bis 4 Kohlenstoff- und 1 bis 3 Halogenatomen steht, und

$X^1$ und $X^2$ gleich oder verschieden sind und für Wasserstoff oder Halogen stehen,

$R^4$ für die Gruppierungen $-O-CO-R^5$ und $-O(S)-R^6$ und ferner für Halogen, Alkyl- und Dialkylamino mit 1 bis 4 Kohlenstoffatomen in jedem Alkylteil steht, wobei

$R^5$ für geradkettiges und verzweigtes Alkyl mit 1 bis 8 Kohlenstoffatomen, für Alkenyl und Alkinyl mit jeweils 2 bis 4 Kohlenstoffatomen, für Halogenalkyl mit 1 bis 4 Kohlenstoff- und 1 bis 5 Halogenatomen steht, ferner für Alkylamino und Dialkylamino mit 1 bis 4 Kohlenstoffatomen in jedem Alkylteil und für gegebenenfalls durch Halogen und Alkyl mit 1 bis 4 Kohlenstoffatomen substituiertes Phenylamino steht,

$R^6$ für Alkyl mit 1 bis 4 und für Alkenyl und Alkinyl mit jeweils 2 bis 4 Kohlenstoffatomen steht und ferner für gegebenenfalls durch Halogen und Alkyl mit 1 bis 4 Kohlenstoffatomen substituiertes Phenyl steht, und

Y für ein Stickstoffatom oder die CH-Gruppe steht,

und deren physiologisch verträglichen Säureadditionssalze und Metallsalzkomplexe gefunden. Sie weisen starke fungizide, insbesondere systemische fungizide Eigenschaften auf.

Die Verbindungen der Formel (I) besitzen zwei asymmetrische Kohlenstoffatome; sie können deshalb in der Erythro- wie in der Threoform vorliegen. In beiden Fällen liegen sie vorwiegend als Racemate vor.

Man erhält die α-Azolylketoderivate der Formel (I), wenn man α-Azolyl-β-hydroxyketone der Formel

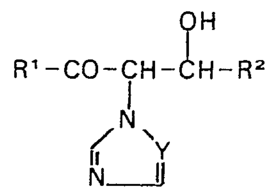 (II)

in welcher

$R^1$, $R^2$ und Y die oben angegebene Bedeutung haben,

a) mit Säurehalogeniden der Formel

$$Hal-CO-R^5 \qquad (III)$$

in welcher

$R^5$ die oben angegebene Bedeutung hat und

Hal für Halogen, insbesondere Chlor oder Brom steht,

gegebenenfalls in Gegenwart eines Lösungsmittels und gegebenenfalls in Gegenwart eines Säurebindemittels umsetzt, oder

b) mit Säureanhydriden der Formel

$$R^5-CO-O-CO-R^5 \qquad (IV)$$

in welcher

$R^5$ die oben angegebene Bedeutung hat,

in Gegenwart eines Lösungsmittels und gegebenenfalls in Gegenwart eines Katalysators umsetzt, oder

c) mit Isocyanaten der Formel

$$O=C=N-R^8 \qquad (V)$$

in welcher

$R^8$ für Alkyl oder gegebenenfalls substituiertes Phenyl steht,

in Gegenwart eines Lösungsmittels und gegebenenfalls in Gegenwart eines Katalysators umsetzt, oder

d) mit einem Halogenierungsmittel, gegebenenfalls in Gegenwart eines Lösungsmittels, umsetzt und gegebenenfalls die dabei erhaltenen α-Azolyl-β-halogenketone der Formel

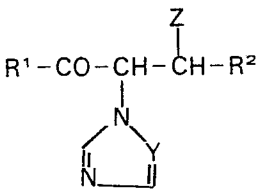 (Ia)

in welcher
$R^1$, $R^2$ und Y die oben angegebene Bedeutung haben und
Z für Halogen steht,

e) mit (Thio)alkoholen der Formel

$$M-O(S)-R^6 \qquad (VI)$$

in welcher
$R^6$ die oben angegebene Bedeutung hat und
M für ein Alkalimetall oder Ammonium steht,

gegebenenfalls in Gegenwart eines Lösungsmittels umsetzt, oder

f) mit Aminen der Formel

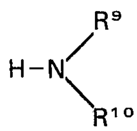 (VII)

in welcher
$R^9$ für Wasserstoff oder Alkyl steht und
$R^{10}$ für Alkyl oder gegebenenfalls substituiertes Phenyl steht,

in Gegenwart eines Lösungsmittels und in Gegenwart eines Säurebinders umsetzt, oder

g) mit Salzen der Formeln

$$M-O-CO-R^5 \qquad (VIII)$$

in welchen
$R^5$ und M die oben angegebene Bedeutung haben,

in Gegenwart eines Lösungsmittels umsetzt.

Weiterhin können die erfindungsgemäß erhältlichen $\alpha$-Azolylketoderivate der Formel (I) durch Umsetzen mit Säuren in die Salze überführt werden, bzw. können durch Reaktion mit Metallsalzen die entsprechenden Metallsalzkomplexe erhalten werden.

Überraschenderweise zeigen die erfindungsgemäßen $\alpha$-Azolylketoderivate eine erheblich höhere fungizide Wirksamkeit, insbesondere bei systemischer Anwendung gegen Schorf- und Mehltauarten, als die aus dem Stand der Technik bekannten substituierten Phenoxytriazolylketo bzw. -hydroxyderivate, welche chemisch und wirkungsmäßig naheliegende Verbindungen sind. Die erfindungsgemäßen Stoffe stellen somit eine Bereicherung der Technik dar.

Ganz besonders bevorzugt sind diejenigen Verbindungen der Formel (I), in denen $R^1$ für tert.-Butyl, sek.-Butyl, Isopropyl, Chlor-tert.-butyl, Brom-tert.-butyl, Fluor-tert.-butyl, 1,3-Dichlor-2-methyl-prop-2-yl, Methylsulfonyloxy-tert.-butyl, Phenylsulfonyloxy-tert.-butyl, 4-Chlorphenylsulfonyloxy-tert.-butyl, Acetoxy-tert.-butyl, Ethylcarbonyloxy-tert.-butyl, Phenylcarbonyloxy-tert.-butyl, 4-Chlorphenylcarbonyloxy-tert.-butyl, Methoxy-tert.-butyl, Ethoxy-tert.-butyl, Isopropoxy-tert.-butyl, Phenoxy-tert.-butyl und 4-Chlorphenoxy-tert.-butyl steht; ferner für Phenyl, Chlorphenyl, Dichlorphenyl, Chlormethylphenyl, Bromphenyl, Nitrophenyl, Biphenylyl, Nitrobiphenylyl, Chlorbiphenylyl, Phenoxyphenyl, Chlorphenoxyphenyl, Benzylphenyl, oder Chlorbenzylphenyl steht; $R^2$ für Trichlormethyl, Dichlorfluormethyl, Dichlormethyl, Chlormethyl, 1,1,2-Tribromethyl, 1,1,2-Trichlorprop-1-yl, Methoxycarbonyl oder Ethoxycarbonyl steht; $R^4$ für Chlor, Brom, Dimethylamino, Ethylmethylamino, Diethylamino, Dipropylamino, Methylamino, Ethylamino, Propylamino, Isopropylamino, Phenylamino, Chlorphenylamino, sowie für die Gruppierungen $-O-CO-R^5$ und $-O(S)-R^6$ steht; $R^5$ und $R^6$ für Methyl, Äthyl, n-Propyl, Isopropyl, n-Butyl, Isobutyl, tert.-Butyl, sek.-Butyl, Allyl, Propargyl, gegebenenfalls einfach oder mehrfach substituiertes Phenyl mit Chlor, Brom oder Methyl als Substituenten stehen; $R^5$ außerdem für Chlormethyl, Dichlormethyl, Methyl- und Ethylamino, Dimethyl- und Diethylamino, Phenylamino oder Chlorphenylamino steht und Y für ein Stickstoffatomen oder die CH-Gruppen steht.

Im einzelnen seien außer den bei den Herstellungsbeispielen genannten Verbindungen die folgenden Verbindungen der allgemeinen Formel (I) genannt:

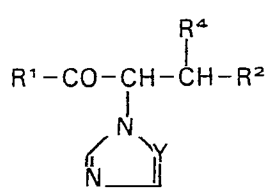

| $R^1$ | $R^2$ | $R^4$ | Y |
|---|---|---|---|
| $C(CH_3)_3$ | $-CCl_2-CHCl-CH_3$ | $-O-CO-CH_3$ | N(CH) |
| $C(CH_3)_3$ | $-CO-O-C_2H_5$ | $-O-CO-CH_3$ | N(CH) |
| $C(CH_3)_3$ | $-CCl_2-CHCl-CH_3$ | $-C-CO-C_2H_5$ | N(CH) |
| $C(CH_3)_3$ | $-CO-O-C_2H_5$ | $-O-CO-C_2H_5$ | N(CH) |
| 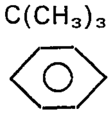 | $-CCl_3$ | $-O-CO-CH_3$ | N(CH) |

| $R^1$ | $R^2$ | $R^4$ | Y |
|---|---|---|---|
| 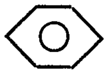 | $-CO-O-C_2H_5$ | $-O-CO-CH_3$ | N(CH) |
| 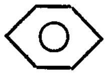 | $-CCl_2-CHCl-CH_3$ | $-O-CO-CH_3$ | N(CH) |
| 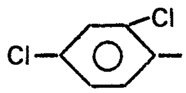 | $-CCl_3$ | $-O-CO-CH_3$ | N(CH) |
| 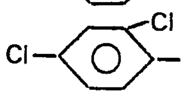 | $-CCl_2-CHCl-CH_3$ | $-O-CO-CH_3$ | N(CH) |
| 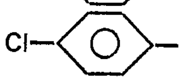 | $-CCl_3$ | $-O-CO-CH_3$ | N(CH) |
| 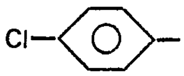 | $-CCl_2-CHCl-CH_3$ | $-O-CO-CH_3$ | N(CH) |
| 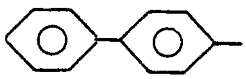 | $-CCl_3$ | $-O-CO-CH_3$ | N(CH) |
| 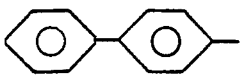 | $-CCl_2-CHCl-CH_3$ | $-O-CO-CH_3$ | N(CH) |
| 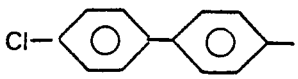 | $-CCl_3$ | $-O-CO-CH_3$ | N(CH) |
| 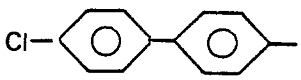 | $-CCl_2-CHCL-CH_3$ | $-O-CO-CH_3$ | N(CH) |
| $ClCH_2-C(CH_3)_2-$ | $-CCl_3$ | $-O-CO-CH_3$ | N(CH) |
| $ClCH_2-O(CH_3)_2-$ | $-CCl_2-CHCl-CH_3$ | $-O-CO-CH_3$ | N(CH) |
| 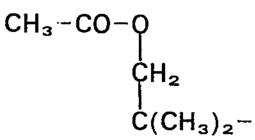 | $-CCl_3$ | $-O-CO-CH_3$ | N(CH) |
| 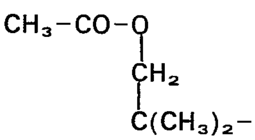 | $-CCl_2-CHCl_2-CH_3$ | $-O-CH-CH_3$ | N(CH) |
| 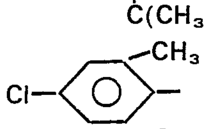 | $-CCl_3$ | $-C-CO-CH_3$ | N(CH) |
| 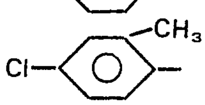 | $-CCl_2-CHCl-CH_3$ | $-O-CO-CH_3$ | N(CH) |
| 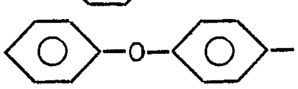 | $-CCl_3$ | $-O-CO-CH_3$ | N(CH) |
| 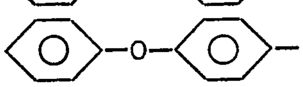 | $-CCl_2-CHCl-CH_3$ | $-O-CO-CH_3$ | N(CH) |
| 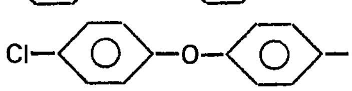 | $-CCl_3$ | $-O-CO-CH_3$ | N(CH) |
| 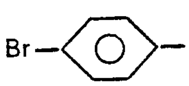 | $-CCl_3$ | $-O-CO-CH_3$ | N(CH) |
| 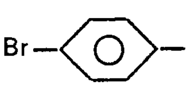 | $-CCl_2-CHCl-CH_3$ | $-O-CO-CH_3$ | N(CH) |

| $R^1$ | $R^2$ | $R^4$ | Y |
|---|---|---|---|
| $CH_3-SO_2-O-CH_2$<br>$C(CH_3)_2-$ | $-CCl_3$ | $-O-CO-CH_3$ | N(CH) |
| $CH_3-SO_2-O-CH_2$<br>$C(CH_3)_2-$ | $-CCl_2-CHCl-CH_3$ | $-O-CO-CH_3$ | N(CH) |
| $BrCH_2-C(CH_3)_2-$ | $-CCl_3$ | $-O-CO-CH_3$ | N(CH) |
| $BrCH_2-C(CH_3)_2-$ | $-CCl_2-CHCl-CH_3$ | $-O-CO-CH_3$ | N(CH) |
| $C(CH_3)_3$ | $-CCl_2-CHCl-CH_3$ | $-O-CO-NHCH_3$ | N(CH) |
| $C(CH_3)_3$ | $-CO-O-C_2H_5$ | $-O-CO-NHCH_3$ | N(CH) |
| 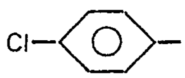 | $-CCl_3$ | $-O-CO-NHCH_3$ | N(CH) |
| 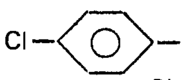 | $-CCl_2-CHCl-CH_3$ | $-O-CO-NHCH_3$ | N(CH) |
| 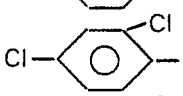 | $-CCl_3$ | $-O-CO-NHC_3$ | N(CH) |
| 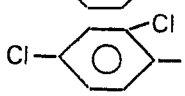 | $-CCl_2-CHCl-CH_3$ | $-O-CO-NHCH_3$ | N(CH) |
| 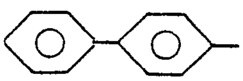 | $-CCl_3$ | $-O-CO-NHCH_3$ | N(CH) |
| 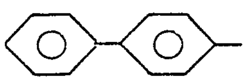 | $-CCl_2-CHCl-CH_3$ | $-O-CO-NHCH_3$ | N(CH) |
| 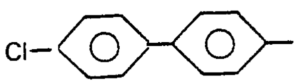 | $-CCl_3$ | $-O-CO-NHCH_3$ | N(CH) |
| 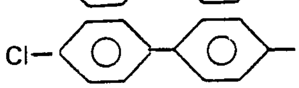 | $-CCl_2-CHCl-CH_3$ | $-O-CO-NHCH_3$ | N(CH) |
| 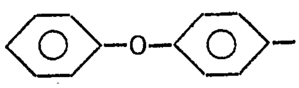 | $-CCl_3$ | $-O-CO-NHCH_3$ | N(CH) |
| 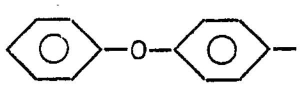 | $-CHCl_2-CHcl-CH_3$ | $-O-CO-NHCH_3$ | N(CH) |
| 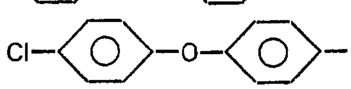 | $-CCl_3$ | $-O-CO-NHCH_3$ | N(CH) |
| 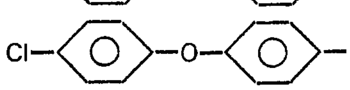 | $-CCl_2-CHCl-CH_3$ | $-O-CO-NHCH_3$ | N(CH) |
| $ClCH_2-C(CH_3)_2-$ | $-CCl_3$ | $-O-CO-NHCH_3$ | N(CH) |
| $ClCH_2-C(CH_3)_2-$ | $-CCl_2-CHCl-CH_3$ | $-O-CO-NHCH_3$ | N(CH) |
| $CH_3-COO-CH_2-C(CH_3)_2-$ | $-CCl_3$ | $-O-CO-NHCH_3$ | N(CH) |
| $CH_3-COO-CH_2-C(CH_3)_2-$ | $-CCl_2-CHCl-CH_3$ | $-O-CO-NHCH_3$ | N(CH) |
| $CH_3-SO_2-CH_2-C(CH_3)_2-$ | $-CCl_3$ | $-O-CO-NHCH_3$ | N(CH) |
| $CH_3-SO_2-CH_2-C(CH_3)_2-$ | $-CCl_2-CHCl-CH_3$ | $-O-CO-NHCH_3$ | N(CH) |
| $CH_3O-C(CH_3)_2-$ | $-CCl_3$ | $-O-CO-NHCH_3$ | N(CH) |
| $CH_3O-C(CH_3)_2-$ | $-CCl_2-CHCl-CH_3$ | $-O-CO-NHCH_3$ | N(CH) |
| $C(CH_3)_3$ | $-CCl_2-CHCl-CH_3$ | Cl | N(CH) |

| $R^1$ | $R^2$ | $R^4$ | Y |
|---|---|---|---|
| $C(CH_3)_3$ | $-CO-O-C_2H_5$ | Cl | N(CH) |
| 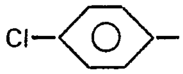 | $-CCl_3$ | Cl | N(CH) |
| 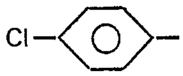 | $-CCl_2-CHCl-CH_3$ | Cl | N(CH) |
| $ClCH_2-C(CH_3)_2-$ | $-CCl_3$ | Cl | N(CH) |
| $ClCH_2-C(CH_3)_2-$ | $-CCl_2-CHCl-CH_3$ | Cl | N(CH) |
| 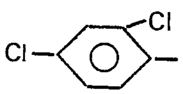 | $-CCl_3$ | Cl | N(CH) |
| 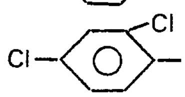 | $-CCl_2-CHCl-CH_3$ | Cl | N(CH) |
| 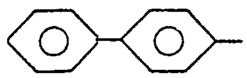 | $-CCl_3$ | Cl | N(CH) |
| 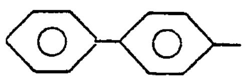 | $-CCl_2-CHCl-CH_3$ | Cl | N(CH) |
| 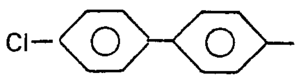 | $-CCl_3$ | Cl | N(CH) |
| 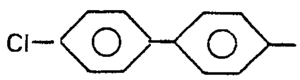 | $-CCl_2-CHCl-CH_3$ | Cl | N(CH) |
| 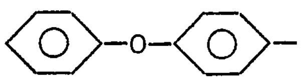 | $-CCl_3$ | Cl | N(CH) |
| 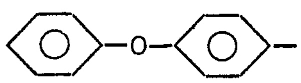 | $-CCl_2-CHCl-CH_3$ | Cl | N(CH) |
| 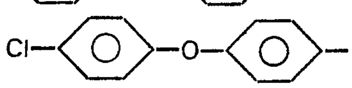 | $-CCl_3$ | Cl | N(CH) |
| 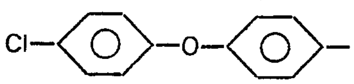 | $-CCl_2-CHCl-CH_3$ | Cl | N(CH) |
| $CH_3-COO-CH_2-C(CH_3)_2-$ | $-CCl_3$ | Cl | N(CH) |
| $CH_3-COO-CH_2-C(CH_3)_2-$ | $-CCl_2-CHCl-CH_3$ | Cl | N(CH) |
| $CH_3-SO_2-CH_2-C(CH_3)_2-$ | $-CCl_3$ | Cl | N(CH) |
| $CH_3-SO_2-CH_2-C(CH_3)_2-$ | $-CCl_2-CHCl-CH_3$ | Cl | N(CH) |
| $CH_3O-C(CH_3)_2-$ | $-CCl_3$ | Cl | N(CH) |
| $CH_3O-C(CH_3)_2-$ | $-CCl_2-CHCl-CH_3$ | Cl | N(CH) |
| $C(CH_3)_3$ | $-CCl_2-CHCl-CH_3$ | $-OCH_3$ | N(CH) |
| $C(CH_3)_3$ | $-CO-O-C_2H_5$ | $-OCH_3$ | N(CH) |
| 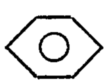 | $-CCl_3$ | $-OCH_3$ | N(CH) |
| 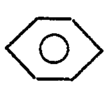 | $-CCl_2-CHCl-CH_3$ | $-OCH_3$ | N(CH) |
| 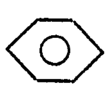 | $-CO-O-C_2H_5$ | $-OCH_3$ | N(CH) |

| $R^1$ | $R^2$ | $R^4$ | Y |
|---|---|---|---|
| 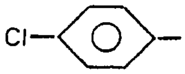 | $-CCl_3$ | $-OCH_3$ | N(CH) |
| 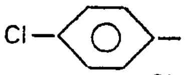 | $-CCl_2-CHCl-CH_3$ | $-OCH_3$ | N(CH) |
| 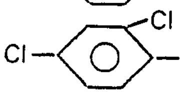 | $-CCl_3$ | $-OCH_3$ | N(CH) |
| 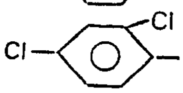 | $-CCl_2-CHCl-CH_3$ | $-OCH_3$ | N(CH) |
| 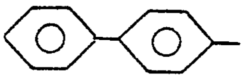 | $-CCl_3$ | $-OCH_3$ | N(CH) |
| 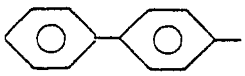 | $-CCl_2-CHCl-CH_3$ | $-OCH_3$ | N(CH) |
| 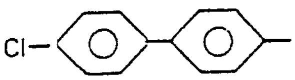 | $-CCl_3$ | $-OCH_3$ | N(CH) |
| 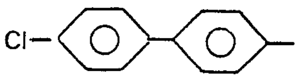 | $-CCl_2-CHCl-CH_3$ | $-OCH_3$ | N(CH) |
| 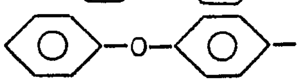 | $-CCl_3$ | $-OCH_3$ | N(CH) |
| 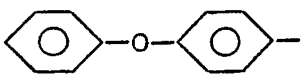 | $-CCl_2-CHCl-CH_3$ | $-OCH_3$ | N(CH) |
| 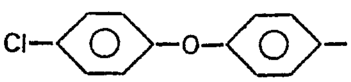 | $-CCl_3$ | $-OCH_3$ | N(CH) |
| 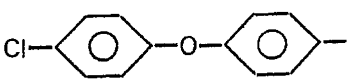 | $-CCl_2-CHCl-CH_3$ | $-OCH_3$ | N(CH) |
| $ClCH_2-C(CH_3)_2-$ | $-CCl_3$ | $-OCH_3$ | N(CH) |
| $ClCH_2-C(CH_3)_2-$ | $-CCl_2-CHCl-CH_3$ | $-OCH_3$ | N(CH) |
| $CH_3-COO-CH_2-C(CH_3)_2-$ | $-CCl_3$ | $-OCH_3$ | N(CH) |
| $CH_3-COO-CH_2-C(CH_3)_2-$ | $-CCl_2-CHCl-CH_3$ | $-OCH_3$ | N(CH) |
| $CH_3-SO_2-CH_2-C(CH_3)_2-$ | $-CCl_3$ | $-OCH_3$ | N(CH) |
| $CH_3-SO_2-CH_2-C(CH_3)_2-$ | $-CCl_2-CHCl-CH_3$ | $-OCH_3$ | N(CH) |
| $CH_3O-C(CH_3)_2-$ | $-CCl_3$ | $-OCH_3$ | N(CH) |
| $CH_3O-C(CH_3)_2-$ | $-CCl_2-CHCl-CH_3$ | $-OCH_3$ | N(CH) |
| $C(CH_3)_3$ | $-CCl_2-CHO-CH_3$ | $-N(C_2H_5)_2$ | N(CH) |
| $C(CH_3)_3$ | $-CO-O-C_2H_5$ | $-N(C_2H_5)_2$ | N(CH) |
| $C(CH_3)_3$ | $-CCl_2-CHCl-CH_3$ | $-NHC_3H_7-n$ | N(CH) |
| $C(CH_3)_3$ | $-CO-O-C_2H_5$ | $-NHC_3H_7-n$ | N(CH) |
| $C(CH_3)_3$ | $-CCl_3$ | $-O-COCH_3$ | CH |
| $C(CH_3)_3$ | $-CCl_3$ | $-O-CO-C_2H_5$ | CH |
| $C(CH_3)_3$ | $-CCl_3$ | $-O-CO-C_3H_7-n$ | CH |
| $C(CH_3)_3$ | $-CCl_3$ | $-O-CO-NHCH_3$ | CH |
| $C(CH_3)_3$ | $-CCl_3$ | Cl | CH |
| $C(CH_3)_3$ | $-CCl_3$ | $-OCH_3$ | CH |

| $R^1$ | $R^2$ | $R^4$ | Y |
|---|---|---|---|
| $C(CH_3)_3$ | $-CCl_3$ | $-OC_2H_5$ | CH |
| $C(CH_3)_3$ | $-CCl_3$ | 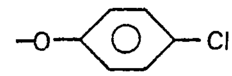 | CH |
| $C(CH_3)_3$ | $-CCl_3$ | $-S-C_2H_5$ | CH |
| $C(CH_3)_3$ | $-CCl_3$ | $-N(C_2H_5)_2$ | CH |
| $C(CH_3)_3$ | $-CCl_3$ | $-NHC_3H_7$-n | CH |
| $C(CH_3)_3$ | $-CCl_3$ | $-S-C_6H_5$ | CH |

Verwendet man beispielsweise 1,1,1-Trichlor-2-hydroxy-3-(1,2,4-triazol-1-yl)-5,5-dimethylhexan-4-on und Dichloracetylchlorid als Ausgangsstoffe, so kann der Reaktionsablauf durch das folgende Formelschema wiedergegeben werden (Verfahren a):

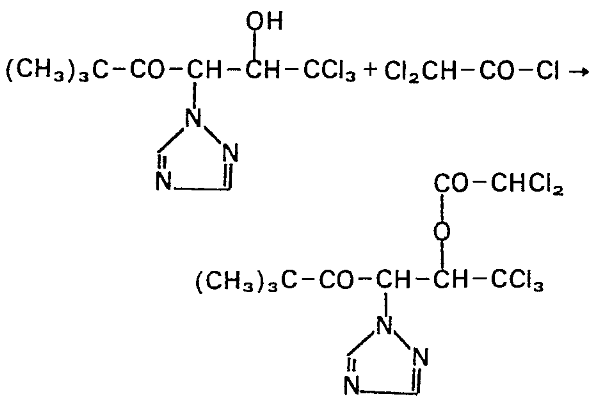

Verwendet man beispielsweise 1,1,1-Trichlor-2-hydroxy-3-(1,2,4-triazol-1-yl)-5,5-dimethylhexan-4-on und Acetanhydrid als Ausgangsstoffe, so kann der Reaktionsablauf durch das folgende Formelschema wiedergegeben werden (Verfahren b):

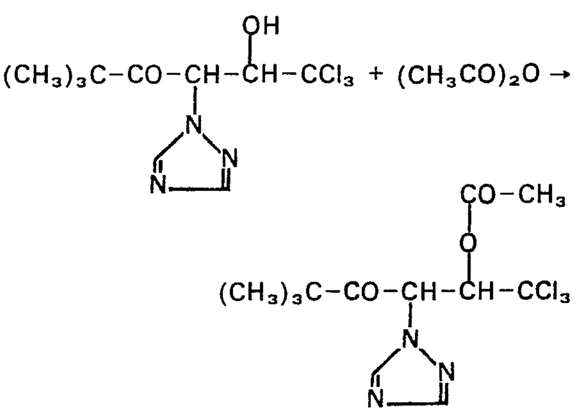

Verwendet man beispielsweise 1,1,1-Trichlor-2-hydroxy-3-(imidazol-1-yl)-5,5-dimethylhexan-4-on und Methylisocyanat als Ausgangsstoffe, so kann der Reaktionsablauf durch das folgende Formelschema wiedergegeben werden (Verfahren c):

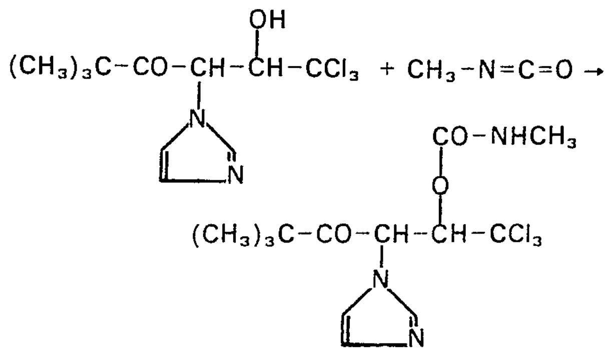

Verwendet man beispielsweise (2,4-Dichlorphenyl)-[3,3,4-trichlor-2-hydroxy-1-(1,2,4-triazol-1-yl)-pentyl]-keton und Trionylchlorid als Ausgangsstoffe, so kann der Reaktionsablauf durch das folgende Formelschema wiedergegeben werden (Verfahren d):

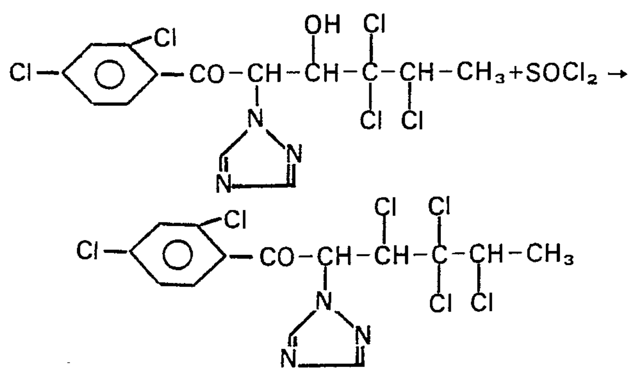

Verwendet man beispielsweise 1,1,1,2-Tetrachlor-3-(1,2,4-triazol-1-yl)-5,5-dimethylhexan-4-on und Natriummethylat als Ausgangsstoffe, so kann der Reaktionsablauf durch das folgende Formelschema wiedergegeben werden (Verfahren e):

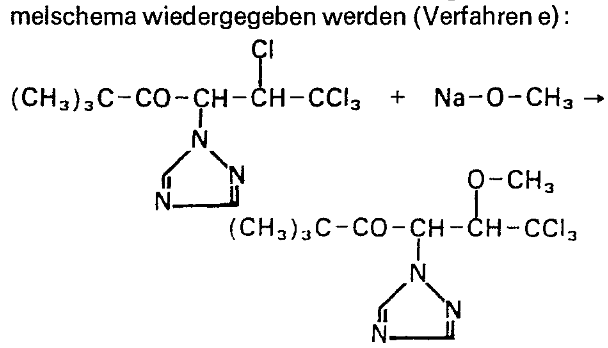

Verwendet man beispielsweise 1,1,1,2-Tetrachlor-3-(1,2,4-triazol-1-yl)-5,5-dimethylhexa-4-on und Diethylamin als Ausgangstoffe, so kann der Reaktionsablauf durch das folgende Formelschema wiedergegeben werden (Verfahren f):

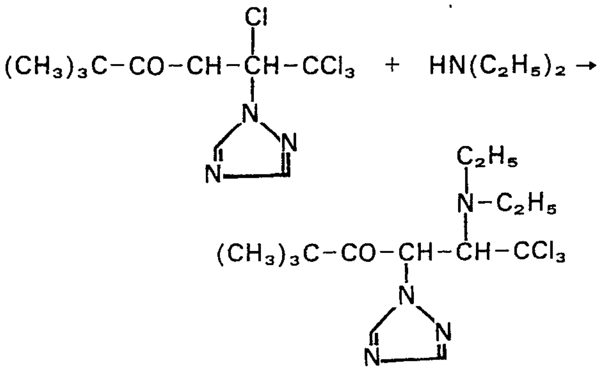

Verwendet man beispielsweise 1,1,1,2-Tetrachlor-3-(1,2,4-triazol-1-yl)-5,5-dimethylhexan-4-on und Natriumacetat als Ausgangsstoffe, so kann der Reaktionsablauf durch das folgende Formelschema wiedergegeben werden (Verfahren g):

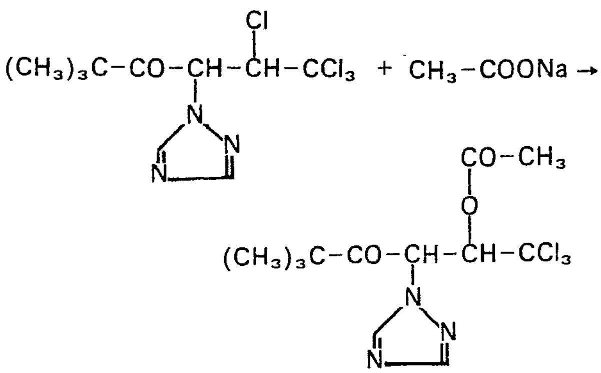

Die für die erfindungsgemäßen Verfahren (a), (b), (c) und (d) als Ausgangsstoffe zu verwendenden α-Azolyl-β-hydroxyketone sind durch die Formel (II) allgemein definiert. In dieser Formel stehen $R^1$, $R^2$ und Y vorzugsweise für die Reste, die bei den Verbindungen der Formel (I) bereits vorzugsweise genannt wurden.

Die α-Azolyl-β-hydroxyketone der Formel (II) sind noch nicht bekannt; sie sind jedoch Gegenstand einer eigenen parallelen Anmeldung, die noch nicht zum Stand der Technik gehört (Deutsche Patentanmeldung P 28 32 233.0 [Le A 18 970] vom 21. Juli 1978). Sie werden erhalten, indem man α-Azolylketone der Formel

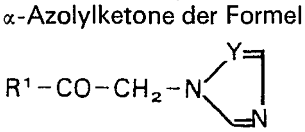 (X)

in welcher

$R^1$ und Y die oben angegebene Bedeutung haben,

mit Aldehyden der Formel

$$R^2-CHO \qquad (XI)$$

in welcher

$R^2$ die oben angegebene Bedeutung hat,

in Gegenwart eines inerten organischen Lösungsmittels, wie beispielsweise Methylenchlorid und insbesondere Eisessig, und in Gegenwart eines Katalysators, wie beispielsweise Titantetrachlorid und insbesondere Natriumacetat, bei Temperaturen zwischen 20 und 100°C umsetzt (vergleiche auch die Herstellungsbeispiele).

Die α-Azolylketone der Formel (X) sind teilweise bekannt (vergleiche DE-OS 2 063 857 und DE-OS 2 431 407 [Le A 15 735]). Die noch nicht bekannten können nach den dort beschriebenen Verfahren erhalten werden, indem man z.B. entsprechende α-Halogenketone mit 1,2,4-Triazol oder Imidazol in Gegenwart eines inerten organischen Lösungsmittels, wie beispielsweise Aceton, und in Gegenwart eines Säurebindemittels, wie beispielsweise Natriumcarbonat, vorzugsweise in der Siedehitze umsetzt (vergleiche auch die Herstellungsbeispiele).

Die für die Herstellung der erfindungsgemäßen Stoffe gemäß Verfahrensvariante (a) außerdem erforderlichen Säurehalogenide sind durch die Formel (III) allgemein definiert. In dieser Formel steht $R^5$ vorzugsweise für die Reste, die bei den Verbindungen der Formel (I) bereits vorzugsweise genannt wurden.

Die Säurehalogenide der Formel (III) sind bekannt oder lassen sich nach üblichen Verfahren herstellen; so z.B. durch Umsetzung von Carbonsäuren bzw. deren Alkalisalzen mit Säurehalogeniden des Phosphors oder Schwefels. Diese Methoden sind aus den allgemeinen Lehrbüchern der organischen Chemie bekannt.

Die weiterhin für die Herstellung der erfindungsgemäßen Stoffe gemäß Verfahrensvariante (b) erforderlichen Säureanhydride sind durch die Formel (IV) allgemein definiert. In dieser Formel steht $R^5$ vorzugsweise für die Reste, die bei den Verbindungen der Formel (I) bereits vorzugsweise genannt wurden.

Die Säureanhydride der Formel (IV) sind bekannt oder lassen sich nach bekannten Verfahren herstellen; so z.B. durch Einwirkung von Säurechloriden auf die Alkalisalze der Carbonsäuren. Diese Verfahren sind allgemein bekannt.

Die außerdem für die Herstellung der erfindungsgemäßen Stoffe gemäß Verfahrensvariante (c) erforderlichen Isocyanate sind durch die Formel (V) allgemein definiert. In dieser Formel steht $R^8$ vorzugsweise für Alkyl mit 1 bis 4 Kohlenstoffatomen sowie für gegebenenfalls substituiertes Phenyl, wobei als Substituenten vorzugsweise infrage kommen: Halogen, Nitro, Cyano, Alkyl mit 1 bis 4 Kohlenstoffatomen, Alkoxy und Alkylthio mit jeweils 1 oder 2 Kohlenstoffatomen und Halogenalkyl mit bis zu 2 Kohlenstoff- und bis zu 5 gleichen oder verschiedenen Halogenatomen, wie insbesondere Fluor- und Chloratomen.

Die Isocyanate der Formel (V) sind bekannt oder lassen sich nach allgemein üblichen und bekannten Verfahren herstellen; so z.B. durch Umsetzen von Aminen mit Phosgen und anschließendem Erhitzen.

Für die Herstellung der erfindungsgemäßen Stoffe gemäß Verfahrensvariante (d) werden außerdem Halogenierungsmittel benötigt. Als solche

seien vorzugsweise anorganische Säurehalogenide genannt, wie Phosphortrichlorid, -tribromid, -pentachlorid, Phosphoroxychlorid, Sulfonyl- und insbesondere Thionylchlorid.

Die ferner für die Herstellung der erfindungemäßen Stoffe gemäß Verfahrensvariante (e) erforderlichen (Thio)alkoholate sind durch die Formel (VI) allgemein definiert. In dieser Formel steht $R^6$ vorzugsweise für die Reste, die bei den Verbindungen der Formel (I) bereits vorzugsweise genannt wurden und M steht vorzugsweise für Natrium, Kalium oder Ammonium.

Die Ausgangsstoffe der Formel (VI) sind allgemein bekannte Verbindungen der organischen Chemie.

Die außerdem für die Herstellung der erfindungsgemäßen Stoffe gemäß Verfahrensvariante (f) erforderlichen Amine sind durch die Formel (VII) allgemein definiert. In dieser Formel steht $R^9$ vorzugsweise für Wasserstoff und Alkyl mit 1 bis 4 Kohlenstoffatomen. $R^{10}$ steht vorzugsweise für Alkyl mit 1 bis 4 Kohlenstoffatomen sowie für gegebenenfalls substituiertes Phenyl, wobei als Substituenten vorzugsweise infrage kommen: Halogen, Cyano, Nitro und Alkyl mit 1 oder 2 Kohlenstoffatomen.

Die Ausgangstoffe der Formel (VII) sind allgemein bekannte Verbindungen der organischen Chemie.

Die weiterhin für die Herstellung der erfindungsgemäßen Stoffe gemäß Verfahrensvariante (g) erforderlichen Salze sind durch die Formel (VIII) allgemein definiert. In dieser Formel steht $R^5$ vorzugsweise für die Reste, die bei den Verbindungen der Formel (I) bereits vorzugsweise genannt wurden. M steht vorzugsweise für die Reste, die bei den (Thio)alkoholaten der Formel (VI) bereits vorzugsweise genannt wurden.

Die Ausgangsstoffe der Formel (VIII) sind allgemein bekannte Verbindungen der organischen Chemie.

Als Lösungsmittel kommen für die Umsetzung gemäß Verfahrensvariante (a) vorzugsweise inerte organische Lösungsmittel infrage. Hierzu gehören vorzugsweise Amide, wie insbesondere Dimethylformamid, sowie Sulfoxide, wie insbesondere Dimethylsulfoxid.

Die Reaktionstemperaturen können bei der Durchführung der Verfahrensvariante (a) in einem größeren Bereich variiert werden. Im allgemeinen arbeitet man zwischen 0 und 100° C, vorzugsweise zwischen 20 und 85° C.

Das erfindungsgemäße Verfahren (a) kann gegebenenfalls in Gegenwart von Säurebindern (Halogenwasserstoffakzeptoren) durchgeführt werden; als solche können alle üblichen Säurebindungsmittel verwendet werden. Hierzu gehören organische Basen, vorzugsweise tertiäre Amine, wie z.B. Triäthylamin, sowie Pyridin oder 4-Dialkylaminopyridin; ferner anorganische Basen, wie z.B. Alkalicarbonate.

Bei der Durchführung der Verfahrensvariante (a) arbeitet man vorzugsweise in molaren Mengen. Die Isolierung der Verbindungen der Formel (I) erfolgt nach üblichen Methoden.

Als Lösungsmittel kommen für die Umsetzung gemäß Verfahrensvariante (b) vorzugsweise die Solventien infrage, die bei der Verfahrensvariante (a) bereits genannt wurden. Insbesondere wird ein Überschuß des jeweils verwendeten Säureanhydrids eingesetzt.

Als Katalysatoren können bei der Verfahrensvariante (b) vorzugsweise alle üblichen sauren Katalysatoren verwendet werden, wie z.B. Schwefelsäure, Chlorwasserstoff, Bromwasserstoff, Bortrifluorid, Zinkchlorid.

Die Reaktionstemperaturen können bei der Durchführung der Verfahrensvariante (b) in einem größeren Bereich variiert werden. Im allgemeinen arbeitet man zwischen 0 und 150° C, vorzugsweise zwischen 80 und 120° C.

Bei der Durchführung der Verfahrensvariante (b) arbeitet man vorzugsweise in molaren Mengen. Der Einfachheit halber wird das eingesetzte Säureanhydrid der Formel (IV) auch als Lösungsmittel verwendet, womit ein entsprechender Überschuß erforderlich wird. Die Isolierung der Verbindungen der Formel (I) erfolgt in üblicher Weise.

Als Lösungsmittel kommen für die Umsetzung gemäß Verfahrensvariante (c) vorzugsweise die Solventien infrage, die bei der Verfahrensvariante (a) bereits genannt wurden. Insbesondere kann auch ein Überschuß des jeweils verwendeten Isocyanats eingesetzt werden.

Als Katalysatoren können beim Verfahren (c) vorzugsweise verwendet werden: tertiäre Basen, wie Triäthylamin und Pyridin oder Zinnorganische Verbindungen, wie Dibutylzinndilaurat.

Die Reaktionstemperaturen können bei der Durchführung der Verfahrensvariante (c) in einem größeren Bereich variiert werden. Im allgemeinen arbeitet man zwischen 0 und 100° C, vorzugsweise zwischen 20 und 80° C.

Bei der Durchführung der Verfahrensvariante (c) arbeitet man vorzugsweise in molaren Mengen. Der Einfachheit halber wird das eingesetzte Isocyanat als Lösungsmittel verwendet, womit ein entsprechender Überschuß erforderlich wird. Zur Isolierung der Verbindungen der Formel (I) wird das Lösungsmittel abdestilliert und der Rückstand nach üblichen Methoden aufgearbeitet.

Als Lösungsmittel kommen für die Umsetzung gemäß Verfahrensvariante (d) vorzugsweise die Solventien infrage, die bei der Verfahrensvariante (a) bereits genannt wurden. Insbesondere kann auch ein Überschuß des jeweils verwendeten anorganischen Säurehalogenids eingesetzt werden.

Die Reaktionstemperaturen können bei der Durchführung der Verfahrensvariante (d) in einem größeren Bereich variiert werden. Im allgemeinen arbeitet man zwischen 0 und 100° C, vorzugsweise zwischen 20 und 80° C.

Bei der Durchführung der Verfahrensvariante (d) arbeitet man vorzugsweise in molaren Mengen. Der Einfachheit halber wird das eingesetzte Halogenierungsmittel auch als Lösungsmittel verwendet, womit ein entsprechender Überschuß erforderlich wird. Die Isolierung der Verbindungen der Formel (I) erfolgt, indem man das überschüssige Halogenierungsmittel z.B. durch Destillation entfernt, das Reaktionsgemisch mit wässriger Natriumhydrogencarbonatlösung versetzt und das Reaktionsprodukt mit einem organischen Solvents ausschüttelt.

Als Lösungsmittel kommen für die Umsetzung gemäß Verfahrensvariante (e) alle inerten organischen Lösungsmittel infrage. Hierzu gehören vorzugsweise Ketone, wie Diethylketon, insbesondere Aceton, Methylisobutylketon und Methylethylketon; Alkohole, wie Methanol, Ethanol oder Isopropanol; Nitrile, wie Propionitril, insbesondere Acetonitril ; aromatische Kohlenwasserstoffe, wie Benzol, Toluol oder Xylol; halogenierte Kohlenwasserstoffe, wie Chloroform oder Methylenchlorid, sowie Formamide, wie insbesondere Dimethylformamid.

Die Reaktionstemperaturen können bei der Durchführung der Verfahrensvariante (e) in einem größeren Bereich variiert werden. Im allgemeinen arbeitet man zwischen 0 und 120° C, vorzugsweise zwischen 20 und 100° C.

Bei der Durchführung der Verfahrensvariante (e) arbeitet man vorzugsweise in molaren Mengen. Zur Isolierung der Verbindungen der Formel (I) wird das Reaktionsgemisch filtriert und das Reaktionsprodukt in üblicher Weise isoliert.

Als Lösungsmittel kommen für die Umsetzung gemäß Verfahrensvariante (f) alle inerten organischen Lösungsmittel infrage. Hierzu gehören vorzugsweise die bei der Verfahrensvariante (e) aufgezählten Solventien.

Das erfindungsgemäße Verfahren (f) wird in Gegenwart eines Säurebinders durchgeführt; als solche können alle üblichen Säurebindungsmittel verwendet werden. Hierzu gehören vorzugsweise die beim Verfahren (a) bereits genannten Stoffe. Es kann aber auch ein entsprechender Überschuß an Amin der Formel (VIII) verwendet werden.

Die Reaktionstemperaturen können bei der Durchführung der Verfahrensvariante (f) in einem größeren Bereich variiert werden. Im allgemeinen arbeitet man zwischen 0 und 100° C, vorzugsweise zwischen 20 und 80° C.

Bei der Durchführung der Verfahrensvariante (f) arbeitet man vorzugsweise in etwa molaren Mengen. Zur Isolierung der Verbindungen der Formel (I) wird das Reaktionsgemisch filtriert und das Reaktionsprodukt in üblicher Weise isoliert.

Als Lösungsmittel kommen für die Umsetzung gemäß Verfahrensvariante (g) vorzugsweise organische Säuren, wie insbesondere Eisessig, Sulfonsäure sowie mit Wasser mischbare inerte organische Lösungsmittel, wie Ketone und Alkohole, infrage.

Die Reaktionstemperaturen können bei der Durchführung der Verfahrensvariante (g) in einem größeren Bereich variiert werden. Im allgemeinen arbeitet man zwischen 0 und 100° C, vorzugsweise zwischen 20 und 80° C.

Bei der Durchführung der Verfahrensvariante (g) setzt man auf 1 Mol der Verbindung der Formel (Ia) etwa 1 bis 2 Mol Salz der Formel (VIII) ein. Die Isolierung der Verbindungen der Formel (I) erfolgt in üblicher Weise.

In einer bevorzugten Ausführungsform der Verfahrensvarianten (e) und (g) wird zweckmäßigerweise so verfahren, daß man von einem (Thio)-alkohol im Fall des Verfahrens (e) und von einem Säurederivat im Fall des Verfahrens (g) ausgeht, letzteres in einem geeigneten inerten organischen Lösungsmittel in das (Thio)alkoholat der Formel (VI) bzw. in ein Salz der Formel (VIII) überführt, und letzteres ohne Isolierung mit einem Halogenid der Formel (Ia) umsetzt, wobei die erfindungsgemäßen Verbindungen der Formel (I) in einem Arbeitsgang erhalten werden.

Nach einer weiteren bevorzugten Ausführungsform werden die Umsetzungen gemäß Verfahrensvarianten (e), (f) und (g) in einem Zweiphasensystem, wie beispielsweise wässrige Natron- oder Kalilauge/Toluol oder Methylenchlorid, unter Zusatz von 0,01 bis 1 Mol eines Phasentransferkatalysators, wie beispielsweise Ammonium- oder Phosphoniumverbindungen, durchgeführt.

Zur Herstellung von Säureadditionssalzen der Verbindungen der Formel (I) kommen alle physiologisch verträglichen Säuren infrage. Hierzu gehören vorzugsweise die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.

Die Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalzkomplexen der Verbindungen der Formel (I) kommen vorzugsweise Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien. Als Anionen der Salze kommen solche in Betracht, die sich von physiologischen Säuren ableiten. Hierzu gehören vorzugsweise die Halogenwasserstoffsäuren, wie z.B. die Chlorwasserstoffsäure und die Bromwasserstoffsäure, ferner Phosphorsäure, Salpetersäure und Schwefelsäure.

Die Metallsalzkomplexe der Verbindungen der Formel (I) können in einfacher Weise nach üblichen Verfahren erhalten werden, so z.B. durch Lösen des Metallsalzes in Alkohol, z.B. Äthanol, und Hinzufügen zur Verbindung der Formel (I). Man kann Metallsalzkomplexe in bekannter Weise, z.B. durch Abfiltrieren, isolieren und gegebenenfalls durch Umkristallisieren reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe haben ein breites Wirkungsspektrum und können angewandt werden gegen parasitäre Pilze, die oberirdische Pflanzenteile befallen und die Pflanzen vom Boden her angreifen, sowie gegen samenübertragbare Krankheitserreger. Eine besonders gute Wirksamkeit entfalten sie gegen parasitäre Pilze auf oberirdischen Pflanzenteilen.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen; so zur Bekämpfung von Erysiphe-Arten, wie z.B. gegen den Erreger des Gurkenmehltaus (Erysiphe cichoracearum) oder des Gersten- bzw. des Getreidemehltaus (Erysiphe graminis); sowie zur Bekämpfung solcher Pilze, die Schorferkrankungen hervorrufen; so zur Bekämpfung von Venturia-Arten, wie z.B. gegen den Erreger des Apfelschorfs (Fusicladium dendriticum). Gute Wirkungen wurden auch gegen Pyricularia und Pellicularia am Reis erzielt. Besonders hervorzuheben ist, daß die erfindungsgemäßen Wirkstoffe nicht nur eine protektive Wirkung entfalten, sondern insbesondere auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebelformulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosoltreibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylenfettsäureester, Polyoxyäthylenfettalkoholäther, z.B. Alkylarylpolyglykoläther, Alkylsulfonate, Alkylsulfate, Arylsulfonate sowie Eiweißhydrolysate; als Dispergiermittel kommen in Frage: z.B. Ligninsulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azolmetallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.% Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet

werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trokkenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.%, vorzugsweise von 0,0001 bis 0,02%, am Wirkungsort erforderlich.

In entsprechenden Aufwandkonzentrationen zeigen die erfindungsgemäßen Stoffe auch akarizide Wirkungen.

Mit nachfolgenden Beispielen werden einige Verwendungsmöglichkeiten eingehender dargestellt:

*Beispiel A*

Erysiphe-Test (Gurken)/Systemisch
Lösungsmittel: 4,7 Gewichtsteile Aceton
Emulgator: 0,3 Gewichtsteile Alkylarylpolyglykoläther
Wasser: 95,0 Gewichtsteile

Man vermischt die für die gewünschte Wirkstoffkonzentration in der Gießflüssigkeit nötige Wirkstoffmenge mit der angegebenen Menge des Lösungsmittels und verdünnt das Konzentrat mit der angegebenen Menge Wasser, welches die genannten Zusätze enthält.

In Einheitserde angezogene Gurkenpflanzen werden im 1- bis 2-Blattstadium innerhalb einer Woche dreimal mit 10 ccm Gießflüssigkeit in der angegebenen Wirkstoffkonzentration, bezogen auf 100 ccm Erde, gegossen.

Die so behandelten Pflanzen werden nach der Behandlung mit Konidien des Pilzes Erysiphe cichoracearum inokuliert. Anschließend werden die Pflanzen bei 23 bis 24°C und einer relativen Luftfeuchtigkeit von 70% im Gewächshaus aufgestellt.

Nach 12 Tagen wird der Befall der Gurkenpflanzen bestimmt. Die erhaltenen Boniturwerte werden in Prozent Befall umgerechnet. 0% bedeutet keinen Befall, 100% bedeutet, daß die Pflanzen vollständig befallen sind.

Wirkstoffe, Wirkstoffkonzentrationen und Ergebnisse werden ermittelt. Bei diesem Test zeigen z.B. folgende Verbindungen eine sehr gute Wirkung, die derjenigen aus dem Stand der Technik bekannten Verbindungen überlegen ist: Verbindungen gemäß Herstellungsbeispielen: 1, 7, 8, 3, 4 und 10.

*Beispiel B*

Fusicladium-Test (Apfel)/Systemisch
Lösungsmittel: 4,7 Gewichtsteile Aceton
Emulgator: 0,3 Gewichtsteile Alkylarylpolyglykoläther
Wasser: 95,0 Gewichtsteile

Man vermischt die für die gewünschte Wirkstoffkonzentration in der Gießflüssigkeit nötige Wirkstoffmenge mit der angegebenen Menge des Lösungsmittels und verdünnt das Konzentrat mit der angegebenen Menge Wasser, welches die genannten Zusätze enthält.

In Einheitserde angezogene Apfelsämlinge werden im 3- bis 4-Blattstadium innerhalb einer Woche einmal mit 10 ccm der Gießflüssigkeit in der angegebenen Wirkstoffkonzentration, bezogen auf 100 ccm Erde, gegossen.

Die so behandelten Pflanzen werden nach der Behandlung mit einer wässrigen Konidiensuspension von Fusicladium dendriticum inokuliert und 18 Stunden lang in einer Feuchtkammer bei 18 bis 20°C und 100% relativer Luftfeuchtigkeit inkubiert. Die Pflanzen kommen dann erneut für 14 Tage ins Gewächshaus.

15 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

Wirkstoffe, Wirkstoffkonzentrationen und Ergebnisse werden ermittelt. Bei diesem Test zeigen z.B. folgende Verbindungen eine sehr gute Wirkung, die derjenigen der aus dem Stand der Technik bekannten Verbindungen überlegen ist: Verbindung gemäß Herstellungsbeispielen: 1, 7, 8, 3, 4, 10, 9 und 6.

*Beispiel C*

Sproßbehandlungstest/Getreidemehltau/protektiv
(blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator (Alkylarylpolyglykoläther) auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var. hordei.

Nach 6 Tagen Verweilzeit der Pflanzen bei einer Temperatur von 21 bis 22°C und einer Luftfeuchtigkeit von 80 bis 90% wertet man den Besatz der Pflanzen mit Mehltaupusteln aus. Der Befallgrad wird in Prozent des Befalls der unbehandelten Kontrollpflanzen ausgedrückt. Dabei bedeutet 0% keinen Befall und 100% den gleichen Befallsgrad wie bei der unbehandelten Kontrolle. Der

Wirkstoff ist umso wirksamer, je geringer der Mehltaubefall ist.

Wirkstoffe, Wirkstoffkonzentrationen in der Spritzbrühe und Befallsgrad werden ermittelt. Bei diesem Test zeigen z.B. folgende Verbindungen eine sehr gute Wirkung, die derjenigen der aus dem Stand der Technik bekannten Verbindungen überlegen ist: Verbindungen gemäß Herstellungsbeispielen: 1, 7, 8, 3 und 10.

*Beispiel D*

Gerstenmehltautest (Erysiphegraminis var. hordei)/systemisch
(pilzliche Getreidesproßkrankheit)

Die Anwendung der Wirkstoffe erfolgt als pulverförmige Saatgutbehandlungsmittel. Sie werden hergestellt durch Abstrecken des jeweiligen Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3×12 Korn in Blumentöpfe 2 cm tief in ein Gemisch aus einem Volumteil Fruhstorfer Einheitserde und einem Volumteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var. hordei bestäubt und bei 21 bis 22°C und 80 bis 90% rel. Luftfeuchte und 16stündiger Belichtung weiter kultiviert. Innerhalb von 6 Tagen bilden sich an den Blättern die typischen Mehltaupusteln aus.

Der Befallsgrad wird in Prozenten des Befalls der unbehandelten Kontrollpflanzen ausgedrückt. So bedeutet 0% keinen Befall und 100% den gleichen Befallsgrad wie bei der unbehandelten Kontrolle. Der Wirkstoff ist um so wirksamer je geringer der Mehltaubefall ist.

Wirkstoffe, Wirkstoffkonzentrationen im Saatgutbehandlungsmittel sowie dessen Aufwandmenge und der prozentuale Mehltaubefall werden ermittelt. Bei diesem Test zeigen z.B. folgende Verbindungen eine sehr gute Wirkung, die derjenigen der aus dem Stand der Technik bekannten Verbindungen überlegen ist: Verbindungen gemäß Herstellungsbeispielen: 1, 3 und 4.

*Herstellungsbeispiele*

*Beispiel 1*

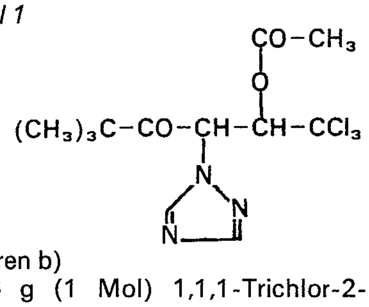

(Verfahren b)

314,6 g (1 Mol) 1,1,1-Trichlor-2-hydroxy-3-(1,2,4-triazol-1-yl)-5,5-dimethylhexan-4-on werden in 1000 ml Essigsäureanhydrid 30 Stunden bei 95°C erhitzt. Anschließend wird das überschüssige Anhydrid im Vakuum abdestilliert, der Rückstand in 750 ml Methylenchlorid aufgenommen und mit 10%iger Natriumhydrogencarbonatlösung neutral gewaschen. Die organische Phase wird über Natriumsulfat getrocknet und durch Abdestillieren des Lösungsmittels im Vakuum eingeengt. Der Rückstand wird mit 150 ml Diisopropyläther verrührt. Man erhält 153,8 g (43% der Theorie) 1,1,1-Trichlor-2-acetoxy-3-(1,2,4-triazol-1-yl)-5,5-dimethylhexan-4-on als reines Diastereomeres der Form A* vom Schmelzpunkt 129 bis 131°C.

(Verfahren a)

Zu 31,5 g (0,1 Mol) 1,1,1-Trichlor-2-hydroxy-3-(1,2,4-triazol-1-yl)-5,5-dimethylhexan-4-on in 100 ml Dimethylformamid werden bei Raumtemperatur 8,0 g (0,1 Mol) Acetylchlorid gegeben. Danach wird 4 Stunden unter Rückfluß erhitzt. Man läßt abkühlen und engt durch Abdestillieren des Lösungsmittels im Vakuum ein. Der Rückstand wird in Methylenchlorid aufgenommen, mit wässriger Natriumhydrogencarbonatlösung gewaschen und über Natriumsulfat getrocknet. Das Lösungsmittel wird abdestilliert und der Rückstand in 100 ml Diisopropyläther aufgenommen. Nach mehrstündigem Stehen fallen farblose Kristalle aus. Man erhält 14,3 g (40% der Theorie) 1,1,1-Trichlor-2-acetoxy-3-(1,2,4-triazol-1-yl)-5,5-dimethylhexan-4-on als reines Diastereomeres der Form A* vom Schmelzpunkt 128 bis 130°C.

(Verfahren g)

16,4 g (0,2 Mol) wasserfreies Natriumacetat werden zu 31,5 g (0,1 Mol) 1,1,1,2-Tetrachlor-3-(1,2,4-triazol-1-yl)-5,5-dimethylhexan-4-on in 150 ml Eisessig gegeben. Man läßt über Nacht bei Raumtemperatur rühren und erhitzt danach ca. 4 Stunden auf 40°C. Anschließend wird das Reaktionsgemisch auf 1000 ml Wasser gegeben und zweimal mit je 250 ml Chloroform extrahiert. Die vereinigten organischen Phasen werden über Natriumsulfat getrocknet und durch Abdestillieren des Lösungsmittels im Vakuum eingeengt. Der feste Rückstand wird in Ethanol umkristallisiert und mit wenig Hexan nachgewaschen. Man erhält 27,8 g (77,8% der Theorie) 1,1,1-Trichlor-2-acetoxy-3-(1,2,4-triazol-1-yl)-5,5-dimethylhexan-4-on als Diastereomerengemisch vom Schmelzpunkt 115 bis 119°C.

*Herstellung der Vorprodukte*

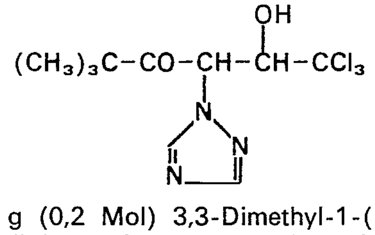

33,4 g (0,2 Mol) 3,3-Dimethyl-1-(1,2,4-triazol-1-yl)-butan-2-on werden in 150 ml Methylenchlorid gelöst und auf −5°C abgekühlt. Dazu tropft man langsam 19 g (0,1 Mol)

Titantetrachlorid, und anschließend 29,5 g (0,2 Mol) Chloral. Während der Zudosierung soll die Innentemperatur bei −5° C konstant bleiben. Danach wird langsam bis zum Rückfluß erwärmt und 3 Stunden verrührt. Die Reaktionslösung wird auf Eis gegossen, und der entstehende weiße käsige Niederschlag abgesaugt. Nach Auskochen in 250 ml Methanol und anschließender Trocknung erhält man 19,9 g (32% der Theorie) 1,1,1-Trichlor-2-hydroxy-3-(1,2,4-triazol-1-yl)-5,5-dimethyl-hexan-4-on vom Schmelzpunkt 220 bis 222° C.

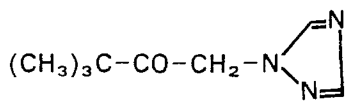

138 g (2 Mol) 1,2,4-Triazol werden bei Raumtemperatur portionsweise zu 276,4 g (2 Mol) gemahlenem Kaliumcarbonat und 269,2 g (2 Mol) α-Chlorpinakolin in 500 ml Aceton gegeben, wobei die Innentemperatur bis zur Siedehitze ansteigt. Man läßt 5 Stunden unter Rückfluß rühren und kühlt dann auf Raumtemperatur ab. Das Reaktionsgemisch wird filtriert und das Filtrat durch Abdestillieren des Lösungsmittels im Vakuum eingeengt. Der ölige Rückstand kristallisiert nach Zugabe von Benzin. Man erhält 240,8 g (72% der Theorie) 3,3-Dimethyl-1-(1,2,4-triazol-1-yl)-butan-2-on vom Schmelzpunkt 62 bis 64° C.

*Beispiel 2*

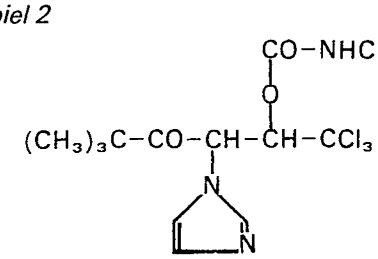

(Verfahren c)

15,8 g (0,05 Mol) 1,1,1-Trichlor-2-hydroxy-3-(imidazol-1-yl)-5,5-dimethylhexan-4-on werden mit 100 ml Methylisocyanat versetzt und 24 Stunden auf 38° C erhitzt. Der entstandene Niederschlag wird abgesaugt und mit 200 ml Chloroform verrührt. Der in Chloroform unlösliche Anteil ist Ausgangsprodukt und wird abgetrennt. Nach Abdestillieren des Chloroforms im Vakuum erhält man 6,9 g (44% der Theorie bezogen auf tatsächlich verbrauchtes Ausgangsprodukt) 1,1,1-Trichlor-2-methylcarbamoyloxy-3-(imidazol-1-yl)-5,5-dimethylhexan-4-on vom Schmelzpunkt 104 bis 108° C.

*Beispiel 3*

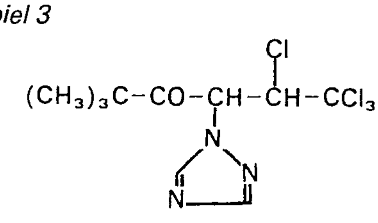

(Verfahren d)

Unter Rühren trägt man 393,3 g (1,25 Mol) 1,1,1-Trichlor-2-hydroxy-3-(1,2,4-triazol-1-yl)-5,5-dimethylhexan-4-on in 750 ml Thionylchlorid ein und erhitzt 36 Stunden auf 60° C. Danach wird das überschüssige Thionylchlorid im Vakuum abdestilliert. Der Rückstand wird in 1,5 l Methylenchlorid aufgenommen, mit 10%iger Natriumhydrogencarbonatlösung neutral gewaschen, über Natriumsulfat getrocknet und durch Abdestillieren des Lösungsmittels im Vakuum eingeengt. Man erhält 408,9 g (98% der Theorie) 1,1,1,2-Tetrachlor-3-(1,2,4-triazol-1-yl)-5,5-dimethylhexan-4-on vom Schmelzpunkt 111 bis 113° C.

*Beispiel 4*

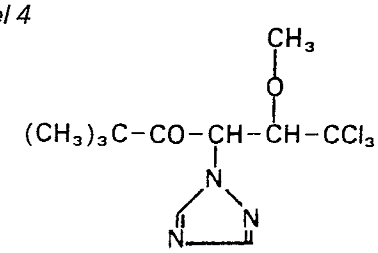

(Verfahren e)

Zu einer Lösung von 33,3 g (0,1 Mol) 1,1,1,2-Tetrachlor-3-(1,2,4-triazol-1-yl)-5,5-dimethylhexan-4-on (Beispiel 3) in 150 ml absolutem Methanol wird eine Lösung von 5,4 g (0,1 Mol) Natriummethylat in 50 ml absolutem Methanol gegeben. Man rührt 5 Stunden bei 60° C nach, saugt das ausgefallene Natriumchlorid ab und engt das Filtrat durch Abdestillieren des Lösungsmittels im Vakuum ein. Der Rückstand wird in 150 ml Methylenchlorid aufgenommen, zweimal mit je 150 ml Wasser gewaschen, über Natriumsulfat getrocknet und eingeengt. Der teilweise kristalline Rückstand wird in 25 ml Diisopropyläther verrührt, wobei er völlig kristallisiert. Nach Umkristallisation aus Isopropanol erhält man 18,8 g (57% der Theorie) 1,1,1-Trichlor-2-methoxy-3-(1,2,4-triazol-1-yl)-5,5-dimethylhexan-4-on vom Schmelzpunkt 93 bis 96° C.

*Beispiel 5*

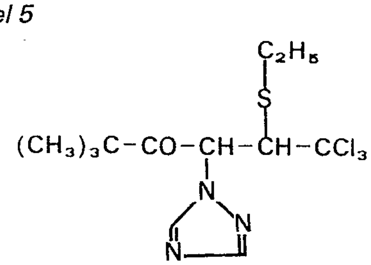

(Verfahren e)

Zu einer Lösung von 3,4 g (0,05 Mol) Natriummethylat in 50 ml absolutem Äthanol werden 3,1 g (0,05 Mol) Ethylmercaptan zugetropft. Man läßt 1 Stunde bei Raumtemperatur nachrühren. Dann tropft man zu diesem Gemisch eine Lösung von 16,7 g 1,1,1,2-Tetrachlor-3-(1,2,4-triazol-1-yl)-5,5-dimethylhexan-4-on (Beispiel 3) in 200 ml Äthanol und läßt 6 Stunden bei Raumtemperatur nachrühren. Anschließend wird das anorganische Salz abgesaugt und das Filtrat eingeengt. Der Rückstand kristallisiert nach dem Verreiben in Hexan. Man erhält 8,9 g (50% der Theorie)

1,1,1-Trichlor-2-äthylmercapto-3-(1,2,4-triazol-1-yl)-5,5-dimethylhexan-4-on vom Schmelzpunkt 101 bis 105°C.

*Beispiel 6*

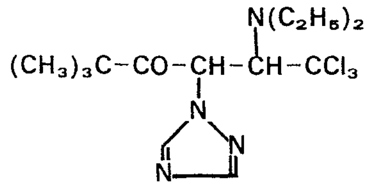

(Verfahren f)

14,6 g (0,2 Mol) Diethylamin werden bei Raumtemperatur langsam zu 33,4 g (0,1 Mol) 1,1,1,2-Tetrachlor-3-(1,2,4-triazol-1-yl)-5,5-dimethylhexan-4-on (Beispiel 3) in 150 ml Tetrahydrofuran getropft. Man läßt anschließend 3 Stunden bei 50 bis 60°C nachrühren. Das ausgefallene Diäthylaminohydrochlorid wird abgesaugt und das Filtrat im Vakuum eingeengt. Nach Dünnschichtchromatographie des Rückstandes über Kieselgel 60 (Merck Korngröße 0,063 bis 0,200 mm) und mit Äther/Hexan im Verhältnis 1:1 als Fließmittel erhält man 1,1,1-Trichlor-2-diäthylamino-3-(1,2,4-triazol-1-yl)-5,5-dimethylhexan-4-on mit 8,9 g (24,1% der Theorie) als reines Diastereomeres der Form A* als gelbes Öl und mit 6,4 g (19,4% der Theorie) als reines Diastereomeres der Form B* vom Schmelzpunkt 103 bis 110°C.

*Formen A und B=jeweils eine der beiden möglichen Isomeren

In entsprechender Weise werden die nachfolgenden Verbindungen der allgemeinen Formel

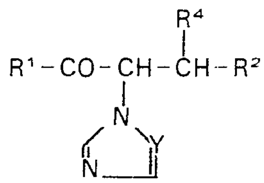 (I)

erhalten:

| Bsp. Nr. | $R^1$ | $R^2$ | $R^4$ | Y | Schmelzpunkt (°C) |
|---|---|---|---|---|---|
| 7 | $(CH_3)_3C-$ | $-CCl_3$ | $-O-CO-C_2H_5$ | N | 115-17 |
| 8 | $(CH_3)_3C-$ | $-CCl_3$ | $-O-CO-C_3H_7-n$ | N | 106-08 |
| 9 | $(CH_3)_3C-$ | $-CCl_3$ | $-O-CO-NHCH_3$ | N | 151-55 |
| 10 | $(CH_3)_3C-$ | $-CCl_3$ | $-O-C_2H_5$ | N | 100-04 |
| 11 | $(CH_3)_3C-$ | $-CCl_3$ | 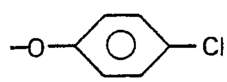 | N | 163-65 |
| 12 | $(CH_3)_3C-$ | $-CCl_3$ | $-NH-C_3H_7-n$ | N | 92-95 |
| 13 | $(CH_3)_3C-$ | $-CCl_3$ | $-O-CO-CH(CH_3)_2$ | N | 93-102 |
| 14 | $(CH_3)_3C-$ | $-CCl_3$ | $-O-CO-NH-$ 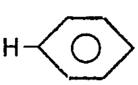 | N | 166-75 |
| 15 | $(CH_3)_3C-$ | $-CCl_3$ | $-S-$ 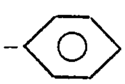 | N | 163-69 |
| 16 | 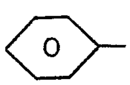 | $-CCl_3$ | $-O-CO-NHCH_3$ | CH | 113-17 |
| 17 | $(CH_3)_3C-$ | $-CCl_3$ | $-O-CO-CH_3$ | N | 226-27 ($xCuCl_2$) |
| 18 | $(CH_3)_3C-$ | $-CCl_3$ | $-O-CO-NHCH_3$ | N | 217-19 ($xCuCl_2$, A-Form) |
| 19 | $(CH_3)_3C-$ | $-CCl_3$ | $-O-CO-NHCH_3$ | N | 230 Zers. ($xCuCl_2$, B-Form) |
| 20 | $ClCH_2-C(CH_3)_2-$ | $-CCl_3$ | $-O-CO-CH_3$ | N | 78-81 |
| 21 | $(CH_3)_3C-$ | $-CCl_3$ | Cl | N | 202-03 ($xCuCl_2$) |

| Bsp. Nr. | $R^1$ | $R^2$ | $R^4$ | Y | Schmelzpunkt (°C) |
|---|---|---|---|---|---|
| 22 | 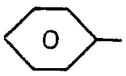 | $-CO-O-C_2H_5$ | $-O-CO-NHCH_3$ | CH | Oel |
| 23 | $(CH_3)_3C-$ | $-CCl_3$ | $-O-CO-NH-$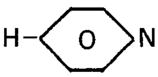 | | 180-82 (xCuCl$_2$, A-Form) |
| 24 | $(CH_3)_3C-$ | $-CCl_3$ | $-O-CO-NH-$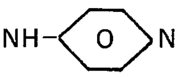 | | 150-58 (xCuCl$_2$, B-Form) |
| 25 | $(CH_3)_3C-$ | $-CCl_2-CHCl-CH_3$ | $-O-CO-CH_3$ | N | 171-74 |
| 26 | $(CH_3)_3C-$ | $-CF_3$ | $-O-CO-CH_3$ | N | 70-79 |
| 27 | $(CH_3)_3C-$ | $-CHCl_2$ | $-O-CO-CH_3$ | N | 109-11 |
| 28 | $FCH_2-C(CH_3)_2-$ | $-CCl_3$ | $-O-CO-CH_3$ | N | 102-04 |
| 29 | $ClCH_2-C(CH_3)_2-$ | $-CCl_3$ | $-O-CO-CH_3$ | N | 78-81 |
| 30 | $(CH_3)_3C-$ | $-CCl_2-CHCl-CH_3$ | $-O-CO-NHCH_3$ | N | 114-15 |
| 31 | $FCH_2-C(CH_3)_2-$ | $-CCl_3$ | $-O-CO-NHCH_3$ | N | 128-29 |
| 32 | $ClCH_2-C(CH_3)_2-$ | $-CCl_3$ | $-O-CO-NHCH_3$ | N | 140-41 |
| 33 | $(CH_3)_3C-$ | $-CHCl_2$ | $-O-CO-NHCH_3$ | N | 159-60 |
| 34 | $(CH_3)_3C-$ | $-CCl_2-CHCl-CH_3$ | Cl | N | Öl |
| 35 | 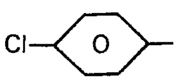 | $-CCl_3$ | Cl | N | 156-70 (x HCl) |

## Patentansprüche

1. α-Azolylketoderivate der allgemeinen Formel

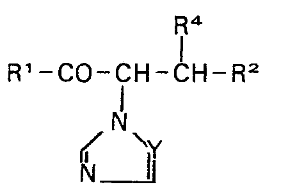

(I)

in welcher

$R^1$ für Alkyl mit 1 bis 4 Kohlenstoffatomen steht, welches gegebenenfalls substituiert sein kann durch Halogen, Alkylcarbonyloxy mit 1 bis 4 Kohlenstoffatomen im Alkylteil, durch Alkylsulfonyloxy mit 1 bis 4 Kohlenstoffatomen, durch Phenylcarbonyloxy- und Phenylsulfonyloxygruppen, wobei letztere noch gegebenenfalls am Phenyl durch Halogen und Alkyl mit 1 bis 2 Kohlenstoffatomen substituiert sein können, durch Alkoxy mit 1 bis 4 Kohlenstoffatomen und ferner noch durch Phenoxy, welches gegebenenfalls noch durch Halogen und Alkyl mit 1 bis 2 Kohlenstoffatomen substituiert sein kann, und endlich noch für Phenyl steht, welches gegebenenfalls substituiert sein kann durch Halogen, Cyano, Nitro und Alkyl mit bis zu 4 Kohlenstoffatomen, ferner durch Cyclohexyl, Halogenalkyl mit bis zu 2 Kohlenstoff- und bis zu 5 Halogenatomen und schließlich durch gegebenenfalls durch Fluor, Chlor, Cyano oder Nitro substituiertes Phenyl, Phenoxy oder Benzyl,

$R^2$ für die Gruppierung $-CX^1X^2R^3$ und für Alkoxycarbonyl mit 1 bis 4 Kohlenstoffatomen im Alkoxyteil steht, wobei

$R^3$ für Halogen, Halogenalkyl mit 1 bis 4 Kohlenstoff- und 1 bis 3 Halogenatomen steht, und

$X^1$ und $X^2$ gleich oder verschieden sind und für Wasserstoff oder Halogen stehen,

$R^4$ für die Gruppierungen $-O-CO-R^5$ und $-O(S)-R^6$ und ferner für Halogen, Alkyl- und Dialkylamino mit 1 bis 4 Kohlenstoffatomen in jedem Alkylteil steht, wobei

$R^5$ für geradkettiges und verzweigtes Alkyl mit 1 bis 8 Kohlenstoffatomen, für Alkenyl und Alkinyl mit jeweils 2 bis 4 Kohlenstoffatomen, für Halogenalkyl mit 1 bis 4 Kohlenstoff- und 1 bis 5 Halogenatomen steht, ferner für Alkylamino und Dialkylamino mit 1 bis 4 Kohlenstoffatomen in jedem Alkylteil und für gegebenenfalls durch Halogen und Alkyl mit 1 bis 4 Kohlenstoffatomen substituiertes Phenylamino steht,

$R^6$ für Alkyl mit 1 bis 4 und für Alkenyl und Alkinyl mit jeweils 2 bis 4 Kohlenstoffatomen steht und ferner für gegebenenfalls durch Halogen

und Alkyl mit 1 bis 4 Kohlenstoffatomen substituiertes Phenyl steht, und

Y für ein Stickstoffatome oder die CH-Gruppe steht,

und deren physiologisch verträglichen Säureadditionssalze und Metallsalzkomplexe.

2. Verfahren zur Herstellung von α-Azolylketoderivaten der allgemeinen Formel

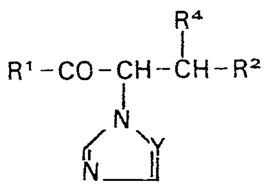

(I)

in welcher

$R^1$ für Alkyl mit 1 bis 4 Kohlenstoffatomen steht, welches gegebenenfalls substituiert sein kann durch Halogen, Alkylcarbonyloxy mit 1 bis 4 Kohlenstoffatomen im Alkylteil, durch Alkylsulfonyloxy mit 1 bis 4 Kohlenstoffatomen, durch Phenylcarbonyloxy- und Phenylsulfonyloxygruppen, wobei letztere noch gegebenenfalls am Phenyl durch Halogen und Alkyl mit 1 bis 2 Kohlenstoffatomen substituiert sein können, durch Alkoxy mit 1 bis 4 Kohlenstoffatomen und ferner noch durch Phenoxy, welches gegebenenfalls durch Halogen und Alkyl mit 1 bis 2 Kohlenstoffatomen substituiert sein kann, und endlich noch für Phenyl steht, welches gegebenenfalls substituiert sein kann durch Halogen, Cyano, Nitro und Alkyl mit bis zu 4 Kohlenstoffatomen, ferner durch Cyclohexyl, Halogenalkyl mit bis zu 2 Kohlenstoff- und bis zu 5 Halogenatomen und schließlich durch gegebenenfalls durch Fluor, Chlor, Cyano oder Nitro substituiertes Phenyl, Phenoxy oder Benzyl,

$R^2$ für die Gruppierung $-CX^1X^2R^3$ und für Alkoxycarbonyl mit 1 bis 4 Kohlenstoffatomen im Alkoxyteil steht, wobei

$R^3$ für Halogen, Halogenalkyl mit 1 bis 4 Kohlenstoff - und 1 bis 3 Halogenatomen steht, und

$X^1$ und $X^2$ gleich oder verschieden sind und für Wasserstoff oder Halogen stehen,

$R^4$ für die Gruppierungen $-O-CO-R^5$ und $-O(S)-R^6$ und ferner für Halogen, Alkyl- und Dialkylamino mit 1 bis 4 Kohlenstoffatomen in jedem Alkylteil steht, wobei

$R^5$ für geradkettiges und verzweigtes Alkyl mit 1 bis 8 Kohlenstoffatomen, für Alkenyl und Alkinyl mit jeweils 2 bis 4 Kohlenstoffatomen, für Halogenalkyl mit 1 bis 4 Kohlenstoff- und 1 bis 5 Halogenatomen steht, ferner für Alkylamino und Dialkylamino mit 1 bis 4 Kohlenstoffatomen in jedem Alkylteil und für gegebenenfalls durch Halogen und Alkyl mit 1 bis 4 Kohlenstoffatomen substituiertes Phenylamino steht,

$R^6$ für Alkyl mit 1 bis 4 und für Alkenyl und Alkinyl mit jeweils 2 bis 4 Kohlenstoffatomen steht und ferner für gegebenenfalls durch Halogen und Alkyl mit 1 bis 4 Kohlenstoffatomen substituiertes Phenyl steht, und

Y für ein Stickstoffatom oder die CH-Gruppe steht,

und deren physiologisch verträglichen Säureadditionssalze und Metallsalzkomplexe, dadurch gekennzeichnet, daß man α-Azolyl-β-hydroxyketone der Formel

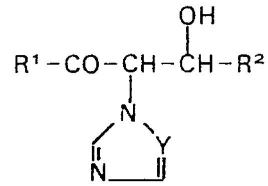

in welcher

$R^1$, $R^2$ und Y die oben angegebene Bedeutung haben,

a) mit Säurehalogeniden der Formel

$$Hal-CO-R^5 \qquad (III)$$

in welcher

$R^5$ die oben angegebene Bedeutung hat und Hal für Halogen, insbesondere Chlor oder Brom steht,

gegebenenfalls in Gegenwart eines Lösungsmittels und gegebenenfalls in Gegenwart eines Säurebindemittels umsetzt, oder

b) mit Säureanhydriden der Formel

$$R^5-CO-O-CO-R^5 \qquad (IV)$$

in welcher

$R^5$ die oben angegebene Bedeutung hat,

in Gegenwart eines Lösungsmittels und gegebenenfalls in Gegenwart eines Katalysators umsetzt, oder

c) mit Isocyanaten der Formel

$$O=C=N-R^7 \qquad (V)$$

in welcher

$R^7$ für Alkyl oder gegebenenfalls substituiertes Phenyl steht,

in Gegenwart eines Lösungsmittels und gegebenenfalls in Gegenwart eines Katalysators umsetzt, oder

d) mit einem Halogenierungsmittel, gegebenenfalls in Gegenwart eines Lösungsmittels, umsetzt und gegebenenfalls die dabei erhaltenen α-Azolyl-β-halogenketone der Formel

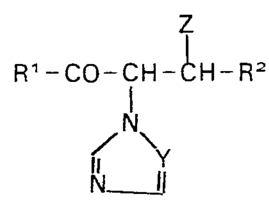

(Ia)

in welcher

$R^1$, $R^2$ und Y die oben angegebene Bedeutung haben und

Z für Halogen steht,

e) mit (Thio)alkoholen der Formel

$$M-O(S)-R^6 \qquad (VI)$$

in welcher

$R^6$ die oben angegebene Bedeutung hat und

M für ein Alkalimetall oder Ammonium steht,

gegebenenfalls in Gegenwart eines Lösungsmittels umsetzt oder

f) mit Aminen der Formel

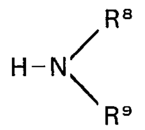

(VII)

in welcher

$R^8$ für Wasserstoff oder Alkyl steht und

$R^9$ für Alkyl oder gegebenenfalls substituiertes Phenyl steht,

in Gegenwart eines Lösungsmittels und in Gegenwart eines Säurebinders umsetzt, oder

g) mit Salzen der Formel

$$M-O-CO-R^5 \qquad (VIII)$$

in welchen

$R^5$ und M die oben angegebene Bedeutung haben,

in Gegenwart eines Lösungsmittels umsetzt, und noch gegebenenfalls die erhaltenen α-Azolylketoderivate durch Umsetzen mit Säuren in die Salze bzw. durch Reaktion mit Metallsalzen in die entsprechenden Metallsalzkomplexe überführt.

3. Fungizide Mittel, gekennzeichnet durch einen Gehalt an mindestens einem α-Azolylketoderivat gemäß Ansprüchen 1 oder 2.

4. Verwendung von α-Azolylketoderivaten gemäß Ansprüchen 1 oder 2 zur Bekämpfung von Pilzen.

## Claims

1. α-Azolylketo derivatives of the general formula

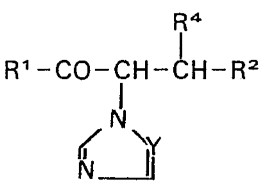

(I)

in which

$R^1$ represents alkyl with 1 to 4 carbon atoms which can optionally be substituted by halogen, alkylcarbonyloxy with 1 to 4 carbon atoms in the alkyl part, by alkylsulphonyloxy with 1 to 4 carbon atoms, by phenylcarbonyloxy and phenylsulphonyloxy groups, it being possible for the latter groups to be also optionally substituted in the phenyl part by halogen and alkyl with 1 to 2 carbon atoms, by alkoxy with 1 to 4 carbon atoms and furthermore also by phenoxy, which can optionally also be substituted by halogen and alkyl with 1 to 2 carbon atoms, and finally also represents phenyl which can optionally be substituted by halogen, cyano, nitro and alkyl with up to 4 carbon atoms, furthermore by cyclohexyl, halogenoalkyl with up to 2 carbon atoms and up to 5 halogen atoms and finally by phenyl, phenoxy or benzyl, optionally substituted by fluorine, chlorine, cyano or nitro,

$R^2$ represents the grouping $-CX^1X^2R^3$ and alkoxycarbonyl with 1 to 4 carbon atoms in the alkoxy part, wherein

$R^3$ represents halogen, halogenoalkyl with 1 to 4 carbon atoms and 1 to 3 halogen atoms, and

$X^1$ and $X^2$ are identical or different and represent hydrogen or halogen,

$R^4$ represents the groupings $-O-CO-R^5$ and $-O(S)-R^6$ and furthermore halogen, alkylamino and dialkylamino with 1 to 4 carbon atoms in each alkyl part, wherein

$R^5$ represents straight-chain and branched alkyl with 1 to 8 carbon atoms, alkenyl and alkinyl with in each case 2 to 4 carbon atoms, halogenoalkyl with 1 to 4 carbon atoms and 1 to 5 halogen atoms, furthermore alkylamino and dialkylamino with 1 to 4 carbon atoms in each alkyl part and phenylamino optionally substituted by halogen and alkyl with 1 to 4 carbon atoms,

$R^6$ represents alkyl with 1 to 4 carbon atoms and alkenyl and alkinyl with in each case 2 to 4 carbon atoms and furthermore represents phenyl optionally substituted by halogen and alkyl with 1 to 4 carbon atoms, and

Y represents a nitrogen atom or the CH group,

and physiologically acceptable acid addition salts and metal salt complexes thereof.

2. Process for the preparation of α-azolylketo derivatives of the general formula

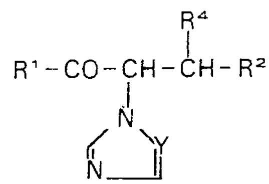

(I)

in which

$R^1$ represents alkyl with 1 to 4 carbon atoms which can optionally be substituted by halogen, alkylcarbonyloxy with 1 to 4 carbon atoms in the alkyl part, by alkylsulphonyloxy with 1 to 4 carbon atoms, by phenylcarbonyloxy and phenylsulphonyloxy groups, it being possible for the latter groups to be also optionally substituted in the phenyl part by halogen and alkyl with 1 to 2 carbon atoms, by alkoxy with 1 to 4 carbon atoms and furthermore also by phenoxy, which can optionally also be substituted by halogen and alkyl with 1 to 2 carbon atoms, and finally also represents phenyl which can optionally be substituted by halogen, cyano, nitro and alkyl with up to 4 carbon atoms, furthermore by cyclohexyl, halogenoalkyl with up to 2 carbon atoms and up to 5 halogen atoms and finally by phenyl, phenoxy or benzyl, optionally substituted by fluorine, chlorine, cyano or nitro,

$R^2$ represents the grouping $-CX^1X^2R^3$ and alkoxycarbonyl with 1 to 4 carbon atoms in the alkoxy part, wherein

$R^3$ represents halogen, halogenoalkyl with 1 to 4 carbon atoms and 1 to 3 halogen atoms, and

$X^1$ and $X^2$ are identical or different and represent hydrogen or halogen,

$R^4$ represents the groupings $-O-CO-R^5$ and

$-O(S)-R^6$ and furthermore halogen, alkylamino and dialkylamino with 1 to 4 carbon atoms in each alkyl part, wherein

$R^5$ represents straight-chain and branched alkyl with 1 to 8 carbon atoms, alkenyl and alkinyl with in each case 2 to 4 carbon atoms, halogenoalkyl with 1 to 4 carbon atoms and 1 to 5 halogen atoms, furthermore alkylamino and dialkylamino with 1 to 4 carbon atoms in each alkyl part and phenylamino optionally substituted by halogen and alkyl with 1 to 4 carbon atoms,

$R^6$ represents alkyl with 1 to 4 carbon atoms and alkenyl and alkinyl with in each case 2 to 4 carbon atoms and furthermore represents phenyl optionally substituted by halogen and alkyl with 1 to 4 carbon atoms, and

Y represents a nitrogen atom or the CH group,

and physiologically acceptable acid addition salts and metal salt complexes thereof, characterised in that α-azolyl-β-hydroxyketones of the formula

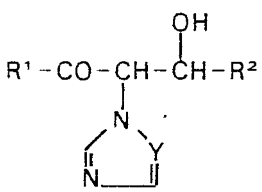

in which

$R^1$, $R^2$ and Y have the meaning indicated above,

a) are reacted with acid halides of the formula

$$Hal-CO-R^5 \qquad (III)$$

in which

$R^5$ has the meaning indicated above and

Hal represents halogen, in particular chlorine or bromine,

if appropriate in the presence of a solvent and if appropriate in the presence of an acid-binding agent, or

b) are reacted with acid anhydrides of the formula

$$R^5-CO-O-CO-R^5 \qquad (IV)$$

in which

$R^5$ has the meaning indicated above,

in the presence of a solvent and if appropriate in the presence of a catalyst, or

c) are reacted with isocyanates of the formula

$$O=C=N-R^7 \qquad (V)$$

in which

$R^7$ represents alkyl or optionally substituted phenyl,

in the presence of a solvent and if appropriate in the presence of a catalyst, or

d) are reacted with a halogenating agent, if appropriate in the presence of a solvent, and, if appropriate, the α-azolyl-β-halogenoketones thereby obtained, of the formula

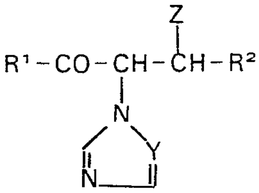

(Ia)

in which

$R^1$, $R^2$ and Y have the meaning indicated above and

Z represents halogen,

e) are reacted with (thio)alcohols of the formula

$$M-O(S)-R^6 \qquad (VI)$$

in which

$R^6$ has the meaning indicated above and

M represents an alkali metal or ammonium,

if appropriate in the presence of a solvent, or

f) are reacted with amines of the formula

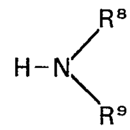

(VIII)

in which

$R^8$ represents hydrogen or alkyl and

$R^9$ represents alkyl or optionally substituted phenyl,

in the presence of a solvent and in the presence of an acid-binding agent, or

g) are reacted with salts of the formula

$$M-O-CO-R^5 \qquad (VIII)$$

in which

$R^5$ and M have the meaning indicated above,

in the presence of a solvent, and the resulting α-azolylketo derivatives are also optionally converted into the salts by reaction with acids, or into the corresponding metal salt complexes by reaction with metal salts.

3. Fungicidal agents, characterised in that they contain at least one α-azolylketo derivative according to Claims 1 or 2.

4. Use of α-azolylketo derivatives according to Claims 1 or 2 for combating fungi.

## Revendications

1. Des dérivés α-azolylcétoniques de formule générale:

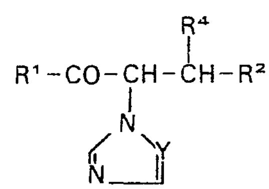

(I)

dans laquelle

$R^1$ est un groupe alkyle ayant 1 à 4 atomes de carbone, qui peut être substitué le cas échéant par un halogène, un radical alkylcarbonyloxy ayant 1 à 4 atomes de carbone dans la partie alkylique, par un radical alkylsulfonyloxy ayant 1 à 4 atomes de carbone, par des groupes phénylcarbonyloxy et phénylsulfonyloxy, ces derniers pouvant encore être substitués le cas échéant sur le radical phényle par un halogène et un radical alkyle ayant 1 ou 2 atomes de carbone, par un radical alkoxy ayant 1 à 4 atomes de carbone et en outre par un radical phénoxy qui peut encore être substitué éventuellement par un halogène et un radical alkyle

ayant 1 ou 2 atomes de carbone, et représente enfin un groupe phényle qui peut être substitué le cas échéant par un halogène, un radical cyano, nitro et alkyle ayant jusqu'à 4 atomes de carbone, en outre par un radical cyclohexyle, halogénalkyle ayant jusqu'à 2 atomes de carbone et jusqu'à 5 atomes d'halogènes et enfin par un radical phényle, phénoxy ou benzyle éventuellement substitué par du fluor, du chlore, un radical cyano ou nitro,

$R^2$ représente le groupement $-CX^1X^2R^3$ et un groupe alkoxycarbonyle ayant 1 à 4 atomes de carbone dans la partie alkoxy,

$R^3$ est un halogène, un groupe halogénalkyle ayant 1 à 4 atomes de carbone et 1 à 3 atomes d'halogènes, et

$X^1$ et $X^2$ sont égaux ou différents et représentent de l'hydrogène ou un halogène,

$R^4$ représente les groupements $-O-CO-R^5$ et $-O(S)-R^6$ et en outre, un halogène, un groupe alkylamino et un groupe dialkylamino ayant 1 à 4 atomes de carbone dans chaque partie alkylique,

$R^5$ est un groupe alkyle à chaîne droite et ramifiée ayant 1 à 8 atomes de carbone, un groupe alcényle et un groupe alcynyle ayant chacun 2 à 4 atomes de carbone, un groupe halogénalkyle ayant 1 à 4 atomes de carbone et 1 à 5 atomes d'halogènes, en outre un groupe alkylamino et un groupe dialkylamino ayant 1 à 4 atomes de carbone dans chaque partie alkylique et un groupe phénylamino éventuellement substitué par un halogène et par un radical alkyle ayant 1 à 4 atomes de carbone,

$R^6$ est un groupe alkyle ayant 1 à 4 atomes de carbone et un groupe alcényle et alcynyle ayant chacun 2 à 4 atomes de carbone et, en outre, un groupe phényle éventuellement substitué par un halogène et un radical alkyle ayant 1 à 4 atomes de carbone, et

Y est un atome d'azote ou le groupe CH,

et leurs sels d'addition d'acides et complexes de sels métalliques acceptables du point de vue physiologique.

2. Procédé de production de dérivés α-azolylcétoniques de formule générale:

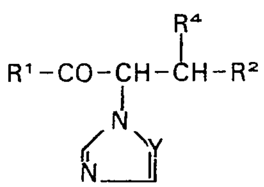

(I)

dans laquelle

$R^1$ est un groupe alkyle ayant 1 à 4 atomes de carbone, qui peut être substitué le cas échéant par un halogène, un radical alkylcarbonyloxy ayant 1 à 4 atomes de carbone dans la partie alkylique, par un radical alkylsulfonyloxy ayant 1 à 4 atomes de carbone, par des groupes phénylcarbonyloxy et phénylsulfonyloxy, ces derniers pouvant encore être substitués le cas échéant sur le radical phényle par un halogène et un radical alkyle ayant 1 ou 2 atomes de carbone, par un radical alkoxy ayant 1 à 4 atomes de carbone et en outre par un radical phénoxy qui peut encore être substitué éventuellement par un halogène et un radical alkyle ayant 1 ou 2 atomes de carbone, et représente enfin un groupe phényle qui peut être substitué le cas échéant par un halogène, un radical cyano, nitro et alkyle ayant jusqu'à 4 atomes de carbone, en outre par un radical cyclohexyle, halogénalkyle ayant jusqu'à 2 atomes de carbone et jusqu'à 5 atomes d'halogènes et enfin par un radical phényle, phénoxy ou benzyle éventuellement substitué par du fluor, du chlore, un radical cyano ou nitro,

$R^2$ représente le groupement $-CX^1X^2R^3$ et un groupe alkoxycarbonyle ayant 1 à 4 atomes de carbone dans la partie alkoxy,

$R^3$ est un halogène, un groupe halogénalkyle ayant 1 à 4 atomes de carbone et 1 à 3 atomes d'halogènes, et

$X^1$ et $X^2$ sont égaux ou différents et représentent de l'hydrogène ou un halogène,

$R^4$ représente les groupements $-O-CO-R^5$ et $-O(S)-R^6$ et en outre, un halogène, un groupe alkylamino et un groupe dialkylamino ayant 1 à 4 atomes de carbone dans chaque partie alkylique,

$R^5$ est un groupe alkyle à chaîne droite et ramifiée ayant 1 à 8 atomes de carbone, un groupe alcényle et un groupe alcynyle ayant chacun 2 à 4 atomes de carbone, un groupe halogénalkyle ayant 1 à 4 atomes de carbone et 1 à 5 atomes d'halogènes, en outre un groupe alkylamino et un groupe dialkylamino ayant 1 à 4 atomes de carbone dans chaque partie alkylique et un groupe phénylamino éventuellement substitué par un halogène et par un radical alkyle ayant 1 à 4 atomes de carbone,

$R^6$ est un groupe alkyle ayant 1 à 4 atomes de carbone et un groupe alcényle et alcynyle ayant chacun 2 à 4 atomes de carbone et, en outre, un groupe phényle éventuellement substitué par un halogène et un radical alkyle ayant 1 à 4 atomes de carbone, et

Y est un atome d'azote ou le groupe CH,

et leurs sels d'addition d'acides et complexes de sels métalliques acceptables du point de vue physiologique, caractérisé en ce qu'on fait réagir des α-azolyl-β-hydroxycétones de formule:

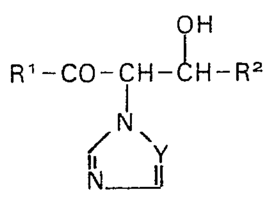

dans laquelle

$R^1$, $R^2$ et Y ont la définition indiquée ci-dessus,

a) avec des halogénures d'acides de formule:

$$Hal-CO-R^5 \qquad (III)$$

dans laquelle

$R^5$ a la définition indiquée ci-dessus, et

Hal est un halogène, en particulier le chlore ou le brome,

éventuellement en présence d'un solvant et le cas échéant en présence d'un accepteur d'acide, ou

b) avec des anhydrides d'acides de formule:

$$R^5-CO-O-CO-R^5 \qquad (IV)$$

dans laquelle

$R^5$ a la définition indiquée ci-dessus,

en présence d'un solvant et le cas échéant en présence d'un catalyseur, ou

c) avec des isocyanates de formule:

$$O=C=N-R^7 \qquad (V)$$

dans laquelle

$R^7$ est un groupe alkyle ou phényle éventuellement substitué,

en présence d'un solvant et le cas échéant en présence d'un catalyseur, ou

d) avec un agent d'halogénation, éventuellement en présence d'un solvant, et le cas échéant les α-azolyl-β-halogénocétones ainsi obtenues de formule:

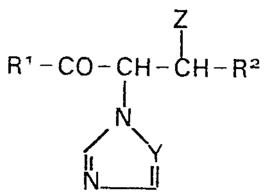

(Ia)

dans laquelle

$R^1$, $R^2$ et Y ont la définition indiquée ci-dessus, et

Z représente un halogène,

e) avec des (thio)-alcools de formule:

$$M-O(S)-R^6 \qquad (VI)$$

dans laquelle

$R^6$ a la définition indiquée ci-dessus, et

M représente un métal alcalin ou l'ion ammonium,

éventuellement en présence d'un solvant, ou

f) avec des amines de formule:

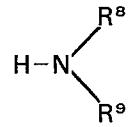

(VII)

dans laquelle

$R^8$ représente l'hydrogène ou un groupe alkyle, et

$R^9$ est un groupe alkyle ou un groupe phényle éventuellement substitué,

en présence d'un solvant et en présence d'un accepteur d'acide, ou

g) avec des sels de formule:

$$M-O-CO-R^5 \qquad (VIII)$$

dans laquelle

$R^5$ et M ont la définition indiquée ci-dessus,

en présence d'un solvant, et on transforme encore le cas échéant les dérivés α-azolylcétoniques obtenus par réaction avec des acides en les sels ou par réaction avec des sels métalliques en les complexes de sels métalliques correspondants.

3. Compositions fongicides, caractérisées par une teneur en au moins un dérivé α-azolylcétonique suivant les revendications 1 ou 2.

4. Utilisation de dérivés α-azolylcétoniques suivant les revendications 1 ou 2 pour la lutte contre des champignons.